# EUROPEAN PATENT SPECIFICATION

(11) **EP 2 106 732 B1**
(45) Date of publication and mention of the grant of the patent: **16.03.2016**
(21) Application number: 08703541.6
(22) Date of filing: 21.01.2008
(51) Int. Cl.: A61B 1/00, A61B 5/07

(54) **DEVICE FOR CHECKING FOR LUMEN PASSAGE AND METHOD OF PRODUCING DEVICE FOR CHECKING FOR LUMEN PASSAGE**
VORRICHTUNG ZUR UNTERSUCHUNG DES LUMENDURCHGANGS UND VERFAHREN ZUR HERSTELLUNG EINER VORRICHTUNG ZUR UNTERSUCHUNG DES LUMENDURCHGANGS
DISPOSITIF PERMETTANT DE CONTRÔLER LE PASSAGE D'UNE LUMIÈRE ET PROCÉDÉ DE PRODUCTION D'UN DISPOSITIF PERMETTANT DE CONTRÔLER LE PASSAGE D'UNE LUMIÈRE

(30) Priority: 30.01.2007 JP 2007019909; 30.01.2007 JP 2007019911
(43) Date of publication of application: 07.10.2009
(73) Proprietor: Olympus Corporation, Shibuya-ku Tokyo (JP)
(72) Inventor: KAWANO, Hironao, Shibuya-ku Tokyo 151-0072 (JP); YOKOI, Takeshi, Shibuya-ku Tokyo 151-0072 (JP)
(74) Representative: von Hellfeld, Axel
(86) International application number: PCT/JP2008/050691
(87) International publication number: WO 2008/093554

(56) References cited:
- JP-A- 2004 248 956
- JP-A- 2005 508 668
- JP-A- 2006 142 013
- US-A- 3 844 285
- US-A1- 2005 063 906
- US-A1- 2006 155 174

## Description

### TECHNICAL FIELD

The present invention relates to a lumen passability checking device that is introduced into the body of a subject to confirm beforehand whether a capsule-type medical device, such as a capsule endoscope, can pass through the hollow organs, such as the small intestine, before the capsule-type medical device is actually used. The present invention also relates to a method of manufacturing a lumen passability checking device.

### BACKGROUND ART

Capsule-type medical devices, such as capsule endoscopes, that are passed through the hollow organs of a subject to observe, examine, perform medical procedures, or provide treatment, have been proposed and put into practical use in recent years. The problem with using such a capsule-type medical device is that, if a relatively narrow hollow organ, such as a small intestine, has an abnormality, e.g., a constricted portion, a swallowed capsule-type medical device resides in the lumen at the constricted portion.

To solve the problem, a lumen passability checking capsule (endoscope pre-test capsule) formed to be the same size and shape as those of a capsule-type medical device is proposed (see, for example, Patent Documents 1 to 4). The lumen passability checking capsule is introduced into a subject to confirm, before the capsule-type medical device is used, whether there is a portion where the capsule-type medical device may reside for an extended time, for example, a constricted portion in the lumen. If the lumen passability checking capsule is discharged normally from the body, it is determined that there is no abnormality, such as a constriction, and a capsule endoscope can be used. If the lumen passability checking capsule is not discharged normally from the body, it is determined that there is an abnormality, such as constriction, and it is inappropriate to use a capsule endoscope.

It is required that a lumen passability checking capsule of this type maintain its original capsule shape for a predetermined time or more in order to confirm whether it cannot pass through the lumen due to, for example, a constriction, and that the lumen passability checking capsule dissolve or decompose and be discharged to prevent it from residing in the body at, for example, a constricted portion after the predetermined time. To satisfy the requirement, various materials have been tried for lumen passability checking capsules. For example, alkali-soluble enteric material, such as (acetic acid/succinic acid) hydro propyl methyl cellulose, which is insoluble in the stomach but soluble in the intestines, is used for components of the lumen passability checking capsule. Compounds of natural polysaccharide and polyalcohol are known as enteric materials as well (see, for example, Patent Documents 5 and 6).
Patent Document 1: Japanese Translation of PCT international application No. 2005-508668
Patent Document 2: Japanese Patent Application Laid-open No. 2004-248956
Patent Document 3: US Patent Application Publication No. 2005/0063906
Patent Document 4: Japanese Patent Application Laid-open No. 2006-142013
Patent Document 5: Japanese Patent Application Laid-open No. H3-232815
Patent Document 6: Japanese Patent Application Laid-open No. H11-49668

US 2005/0063906 A1 relates to a device and method which is described for enabling an examining device to pass through an abnormally configured body lumen. The device may pass through a body lumen while maintaining an initial dimension. Upon encounter with an abnormally configured body lumen, the device may be depleted and take on a final dimension after a predetermined time period, regardless of its current orientation with respect to the body lumen, so that the device may pass through and vacate the abnormally configured body lumen shortly after the predetermined time period may have elapsed.

### DISCLOSURE OF INVENTION

### PROBLEM TO BE SOLVED BY THE INVENTION

According to Patent Documents 1 and 2, it is desirable that the time taken by a lumen passability checking capsule to completely dissolve (time during which the capsule shape can be maintained) be set to, for example, 100 hours or more, not less than one day, or not more than seven days. The dissolution time depends on the enteric material, and it is difficult to control the dissolution time by depending on the material. The time taken by the enteric material, which is described above as an example, to dissolve is about one day at maximum because of the size of the lumen passability checking capsule. A desired time cannot be assured. Therefore, the predetermined time necessary to confirm patency cannot be assured for every subject and patency therefore cannot be definitely confirmed.

The present invention is made in view of the above problem, and an object of the present invention is to provide a lumen passability checking device that can definitely maintain its capsule shape for a predetermined time necessary to confirm patency, and a method of manufacturing a lumen passability checking device.

### MEANS FOR SOLVING PROBLEM

A lumen passability checking device according to the present invention is defined by independent claim 1.

Specific embodiments of the lumen passability checking device are defined by dependent claims 2 to 4.

A method according to the present invention for manufacturing a lumen capsule confirmation device is defined by independent claim 5.

Specific embodiments of the method are defined by dependent claims 6 and 7.

### EFFECT OF THE INVENTION

In the lumen passability checking device according to the present invention, the soluble portion provided in the first insoluble portion, which maintains the capsule shape, is divided with the second insoluble portion, so that the dissolution route that progresses from a part of the surface to the inside is formed. Making the dissolution route longer structurally makes longer the time taken by the soluble portion to dissolve in the lumen. This maintains the capsule shape for an extended time, i.e., maintain the capsule shape for a predetermined time necessary for confirming patency, which makes it possible to definitely confirm patency of the lumen.

In the lumen passability checking device according to the present invention, the second insoluble portion, which forms the dissolution route, is provided to extend toward the center of the capsule shaped body. Thus, the dissolution route can be formed in which the soluble portion dissolves from the center portion of the capsule shaped body to the outside. This makes longer the time taken by the soluble portion near the first insoluble portion forming the outer surface of the capsule shaped body to dissolve, which leads to an effect that the capsule shape can be maintained for an extended time.

In the lumen passability checking device according to the present invention, the dissolution route is further divided and compartmentalized by the third insoluble portion. Thus, the dissolution route is made longer, which leads to an effect that the capsule shape can be maintained for a more extended time.

In the lumen passability checking device according to the present invention, the end of the second insoluble portion is connected to the end of the first insoluble portion. Thus, the continuity between the first insoluble portion and the second insoluble portion can be maintained on the outer surface of the capsule shaped body. Therefore, the capsule shaped body does not collapse from the end of the first insoluble portion, which leads to an effect that the capsule shape can be maintained for an extended time.

In the lumen passability checking capsule device according to the present invention, the second insoluble portion is provided in a layer approximately parallel to the first insoluble portion. Therefore, the dissolution route can be formed in a large area in the circumferential direction of the capsule shaped body. This makes longer the time until the soluble portion in the dissolution route dissolves, which leads to an effect that the capsule shape can be maintained for an extended time.

In the lumen passability checking device and the method of manufacturing a lumen passability checking device according to the present invention, the through hole that penetrates through the center portion of the capsule shaped body is provided beforehand in the lumen passability checking device, which has the capsule shape and the size capable of being introduced into the body and loses the original capsule shape with time because the lumen passability checking device resides in the lumen. The fluid path is formed such that the anterior and posterior portions of the lumen are communicated with each other when the original capsule shape closely contacts with the inner surface of the lumen in the body and blocks the lumen. Thus, the portions of the lumen, which are anterior and posterior to the lumen passability checking device, are communicated with each other, which prevents, for example, blockage in, for example, a constricted portion.

### BRIEF DESCRIPTION OF DRAWINGS

FIG. 1 is a schematic perspective view of a configuration example of a lumen passability checking capsule according to a first example useful for understanding the present invention;
FIG. 2 is a center sectional side view of FIG. 1;
FIG. 3 is a schematic sectional side view of a lumen passability checking capsule of Modification 1;
FIG. 4 is a schematic perspective view of a lumen passability checking capsule of Modification 2;
FIG. 5 is a schematic perspective view of a lumen passability checking capsule of Modification 3;
FIG. 6 is an end surface side view of FIG. 5;
FIG. 7 is a schematic perspective view of a configuration example of a lumen passability checking capsule according to a second example useful for understanding the present invention;
FIG. 8 is a center sectional side view of FIG. 7;
FIG. 9 is a schematic perspective view of a lumen passability checking capsule of Modification 4;
FIG. 10 is a center sectional side view of a lumen passability checking capsule of Modification 5;
FIG. 11 is a cross-sectional view representing how a divided insoluble portion deforms;
FIG. 12 is a center sectional side view of a lumen passability checking capsule of Modification 6;
FIG. 13 is a center sectional front view of a configuration example of a lumen passability checking capsule according to a third example useful for understanding the present invention;
FIG. 14 is a partial cross-sectional view of a lumen passability checking capsule of Modification 7;
FIG. 15 is a sectional front view of a lumen passability checking capsule according to a fourth example useful for understanding the present invention;
FIG. 16 is a sectional front view of a lumen passability checking capsule of Modification 8;
FIG. 17 is a sectional front view of a configuration example of a lumen passability checking capsule according to a first embodiment of the present invention;
FIG. 18 is a side view of FIG. 17;
FIG. 19 is a sectional front view of a configuration example of a lumen passability checking capsule according to a fifth example useful for understanding the present invention;
FIG. 20 is a sectional front view of a configuration example of a lumen passability checking capsule of Modification 9;
FIG. 21 is a sectional front view of a configuration example of a lumen passability checking capsule according to a sixth example useful for understanding the present invention;
FIG. 22 is a center portion sectional side view of a configuration example of lumen passability checking capsule according to a seventh example useful for understanding the present invention;
FIG. 23 is a front view of FIG. 22;
FIG. 24 is a center portion sectional side view of a lumen passability checking capsule of Modification 10;
FIG. 25 is a schematic sectional side view of a configuration example of a lumen passability checking capsule according to a second embodiment of the present invention;
FIG. 26 is a center sectional side view of FIG. 25;
FIG. 27 is a schematic diagram representing a state where the lumen passability checking capsule shown in FIG. 25 is stuck at a constricted portion;
FIG. 28 is a schematic sectional side view of a lumen passability checking capsule of Modification 11;
FIG. 29 is a schematic sectional side view of a lumen passability checking capsule of Modification 12;
FIG. 30 is a schematic sectional side view of a lumen passability checking capsule of Modification 13;
FIG. 31 is a schematic sectional side view of a lumen passability checking capsule of Modification 14;
FIG. 32 is a center sectional side view of FIG. 31;
FIG. 33 is a schematic sectional side view of a configuration example of a lumen passability checking capsule according to a third embodiment of the present invention;
FIG. 34 is a center sectional side view of FIG. 33;
FIG. 35 is a schematic sectional side view of a lumen passability checking capsule of Modification 15;
FIG. 36 is a schematic sectional side view of a configuration example of a lumen passability checking capsule according to an fourth embodiment of the present invention;
FIG. 37 is a center sectional side view of FIG. 36;
FIG. 38 is a center sectional side view of a configuration example of a lumen passability checking capsule according to a fifth embodiment of the present invention;
FIG. 39 is a center sectional side view of a configuration example of a lumen passability checking capsule of Modification 16;
FIG. 40 is a schematic sectional side view of a configuration example of a lumen passability checking capsule according to a sixth embodiment of the present invention;
FIG. 41 is a schematic perspective view of a configuration example of a lumen passability checking capsule according to a seventh embodiment of the present invention; and
FIG. 42 is a center sectional side view of a configuration example of a lumen passability checking capsule according to a eighth embodiment of the present invention.

### EXPLANATIONS OF LETTERS OR NUMERALS

10, 10A, 10B, 10C, 20, 20A, 20B, 20C, 30, 40, 40A, 50, 60, 60A, 70, 80, 80A LUMEN PASSABILITY CHECKING CAPSULE
11a, 11b CONVEX PORTION
12, 16, 18, 42, 63, 64, 83 INSOLUBLE PORTION
13, 14, 41 SOLUBLE PORTION
15, 15a, 15b IDENTIFIED PORTION
17, 62, 82 DISSOLUTION ROUTE
42a, 63a END
43, 61, 81 OPENING
110, 110A, 110B, 110C, 110D, 120, 120A, 130, 140, 150, 160, 170, 180
LUMEN PASSABILITY CHECKING CAPSULE
111a, 111b CONVEX PORTION
112, 112a to 112d, 122, 182 THROUGH HOLE.
113, 113a, 115a, 115b, 123, 163 SOLUBLE PORTION 114, 114a to 114c, 124, 126, 127 INSOLUBLE PORTION
116, 116a, 116b, 166 IDENTIFIED PORTION
125 OPENING
124a, 126a END
132 COMMUNICATING HOLE
141 GROOVE
151 PROTRUDING PORTION
174 SHEET MEMBER
200 LUMEN
201 CONSTRICTED PORTION

### BEST MODE(S) FOR CARRYING OUT THE INVENTION

Lumen passability checking capsules and methods of manufacturing a lumen passability checking capsule, which are preferred embodiments of a lumen passability checking device and a method of manufacturing a lumen passability checking device according to the present invention, are explained below with reference to the accompanying drawings. Lumen passability checking capsules of each embodiment and modification is explained, taking examples where they are used for a lumen of, for example, a stomach and intestines, where patency is confirmed. In the drawings, identical portions or corresponding portions are denoted by the same reference numerals.

### (First example)

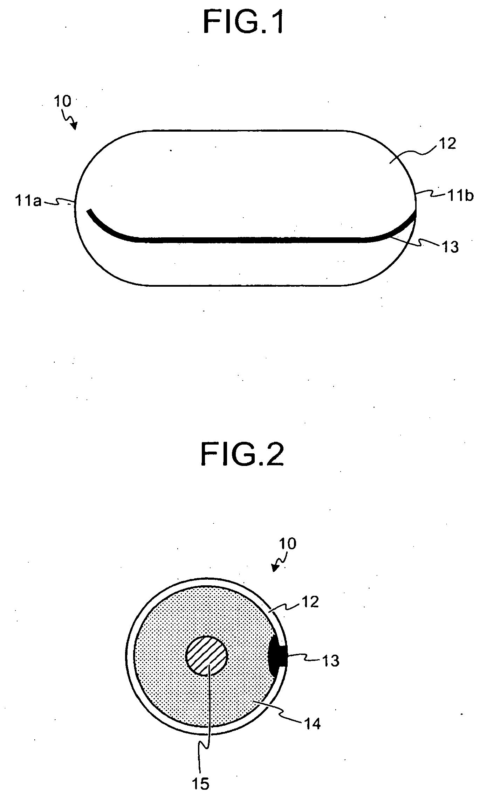

A lumen passage capsule according to a first example useful for understanding the present invention is explained below with reference to FIGs. 1 and 2. FIG. 1 is a schematic perspective view of a configuration example of a lumen passability checking capsule 10 according to the first example, and FIG. 2 is a center sectional side view of the lumen passability checking capsule 10.

Schematically, the lumen passability checking capsule 10 according to the first example has a shape and a size approximately same as those of a capsule-type endoscope aimed at internal examination and observation of a lumen of, for example, intestines and a stomach, and can be introduced into the body of a subject. The lumen passability checking capsule 10 includes a domed-capsule shape having semi-sphere, i.e., domed, convex portions 11a and 11b on both ends in the longitudinal direction. The lumen passability checking capsule 10, which has such a domed-capsule shape, includes an insoluble portion 12, a soluble portion 13, a second soluble portion 14, and an identified portion 15.

The insoluble portion 12 is made of a material, such as flexible metal or resin, that is basically insoluble in the body and impermeable (impenetrable) to body fluids. The insoluble portion 12 is a thin portion that forms a surface layer of the domed-capsule shape, excluding a part of the surface layer, and that forms a structure that forms an outer shape of the domed-capsule shape. The soluble portion 13 is made of a material soluble in the body, and is provided to a portion linearly formed on the surface layer of the domed-capsule shape such that, when the soluble portion 13 dissolves, the insoluble portion 12 deforms in a direction such that the insoluble portion 12 becomes smaller. In other words, a linearly missing portion is formed in a portion of the insoluble portion 12 such that the rigidity cannot be maintained. The missing portion is filled with the soluble portion 13 to assure the rigidity, so that the surface layer that maintains the domed-capsule shape is formed. In the first example, the soluble portion 13 is formed linearly in the longitudinal direction of the domed-capsule shape at least between both of the convex portions 11a and 11b on both ends of the domed-capsule shape in the longitudinal direction. The soluble portion 13 is provided such that it not only fills the missing portion but also slightly edges into the surface of an inner wall near the missing portion of the insoluble portion 12, so that a predetermined time (for example, one to two days or more) is required for dissolution of the soluble portion 13.

The second soluble portion 14 is provided inside the insoluble portion 12, which forms the surface layer, and serves as contents of the lumen passability checking capsule 10 such that the second soluble portion 14 dissolves after the soluble portion 13 dissolves. The second soluble portion 14 consists of a material that has solubility higher than that of the soluble portion 13. In the first example, the soluble portion 13 is formed of an enteric material that does not dissolve easily in the stomach but dissolves while the lumen passability checking capsule 10 passes through the small intestine and the large intestine. For example, the soluble portion 13 is formed of an alkali-soluble enteric material, such as (acetic acid/succinic acid) hydro propyl methyl cellulose that is disclosed in Patent Document 3, or a natural polysaccharide-polyalcohol compound that is disclosed in Patent Documents 5 and 6, and that is obtained by uniformly mixing natural polysaccharide, which is selected from carrageenan, alginic acid, alginate, alginate derivative, agar, locust bean gum, guar gum, amylopectin, pectin, xanthan gum, glucomannan, chitin, and pullulan into any one selected from polyalcohol, sugar alcohol, disaccharide, trisaccharide, and oligosaccharide. In contrast, the second soluble portion 14 is formed of a soluble material that easily dissolves, such as a material that is mainly composed of chitosan, ceratin, or cellulose that is natural macromolecules.

The identified portion 15 is identified by an identifying device outside the subject, so that the position where the lumen passability checking capsule 10 resides or where it currently passes through is confirmed. The identified portion 15 is a radiopaque member, for example, barium sulfate, metal (gold, titanium, or stainless steel), or an electronic tag, such as an RF-ID (Radio Frequency Identification) tag. If the identified portion 15 is a radiopaque member, such as barium sulfate or metal, it can be confirmed whether the lumen passability checking capsule 10 resides in, for example, a constricted portion by scoping the body of the subject using a fluoroscope and confirming whether there is the radiopaque member. If the identified portion 15 is an electric tag, such as an RF-ID tag, electric power is sent to the electric tag using a tag reader-writer to operate an IC chip. By receiving necessary information from the tag, the presence of the tag can be confirmed. A member that has a size such that it can pass through even a constricted portion is used for the identified portion 15, and the identified portion 15 is arranged in the second soluble portion 14 and in a center portion of the lumen passability checking capsule 10. However, the position is not particularly limited to the center portion. If the insoluble portion 12 that serves as the surface layer is metallic, the insoluble portion 12 is used as the identified portion 15 and it is unnecessary to provide the identified portion 15 in the second soluble portion 14.

The lumen passability checking capsule 10 is formed by, for example, filling the second soluble portion 14 including the identified portion 15 into the inside of the insoluble portion 12 having a missing portion in a part of the domed-capsule body, and filling the soluble portion 13 along the missing portion.

The subject introduces the lumen passability checking capsule 10 having the above configuration is introduced into the body from the mouth a predetermined time (for example, few days) prior to internal examination and observation of the lumen using a capsule endoscope to be adopted, to previously confirm whether the capsule endoscope can pass through the hollow organs, such as the small intestine.

The lumen passability checking capsule 10, which is introduced into the body, has the size and shape same as those of the capsule endoscope, passes through the lumen of, for example, the stomach, the small intestine, and the large intestine, sequentially by peristalsis of the lumen and finally discharged to the outside of the body if there is no abnormal portion that is extremely narrowed due to, for example, a constricted portion in the lumen. Because the surface layer of the lumen passability checking capsule 10 is mainly formed as the insoluble portion 12 and the soluble portion 13, which is linear and exposed to the outside as a part of the surface layer, is formed of a enteric material, the lumen passability checking capsule 10 moves forward without dissolution in body fluids, such as gastric juice until it passes through the stomach. In the process of passing through the small intestine and the large intestine, the lumen passability checking capsule 10 moves forward while the soluble portion 13, which is made of a enteric material, gradually dissolves. For a predetermined time until the soluble portion 13 dissolves, the domed-capsule shape is maintained. If there is no abnormality, such as a constricted portion, in the small intestine, the lumen passage conformation capsule 10 moves forward without residing in the small intestine and the large intestine while maintaining the domed-capsule shape. Thus, it is confirmed that there is no abnormality, such as a constricted portion, in the lumen, i.e., that the original capsule endoscope can pass through smoothly.

In contrast, if there is an abnormality, such as a constricted portion, in a portion, for example, the small intestine, the lumen passability checking capsule 10 is inhibited from moving forward even with peristalsis, and resides in the constricted portion. As the residing time elapses, the dissolution of the soluble portion 13, which is linear, progresses. The lumen passability checking capsule 10 dissolves completely when a predetermined time elapses, and the second soluble portion 14 is exposed to the outside and dissolution of the second soluble portion 14 starts. Because the second soluble portion 14 is made of a material having solubility higher than that of the soluble portion 13, the second soluble portion 14 dissolves in a short time and flows into the small intestine from the missing portion of the insoluble portion 12, and the insoluble portion 12 becomes hollow in a short time. Because the soluble-portion 13 is missing, the rigidity, for maintaining the domed-capsule shape, of the insoluble portion 12 that resides in the lumen passability checking capsule 10 thus hollowed lowers and the insoluble portion 12 easily deforms in a direction such that the insoluble portion 12 becomes smaller. Due to the peristalsis of the small intestine, the insoluble portion 12 crumples such that the outer diameter of the insoluble portion 12 becomes smaller. Thus, even when the lumen passability checking capsule 10 cannot pass through the small intestine in a predetermined time and resides in, for example, a constricted portion, the lumen passability checking capsule 10 crumples and becomes smaller after the predetermined time. This definitely allows the insoluble portion 12 to pass through the constricted portion and be discharged, so that it does not reside in the lumen. Specifically, the soluble portion 13 is formed linearly in the longitudinal direction between both ends of the domed-capsule shape in the longitudinal direction in the first example; therefore, when the soluble portion 13 dissolves, the insoluble portion 12, which forms the domed-capsule shape, easily crumples in a radial direction, so that the diameter thereof in the moving direction becomes smaller. This increases the passage performance in, for example, a constricted portion.

After the predetermined time or more elapses, the identifying device outside the body of the subject identifies the identified portion 15 to reconfirm whether the lumen passability checking capsule 10 resides in the lumen after the predetermined time or more elapses.

### (Modification 1)

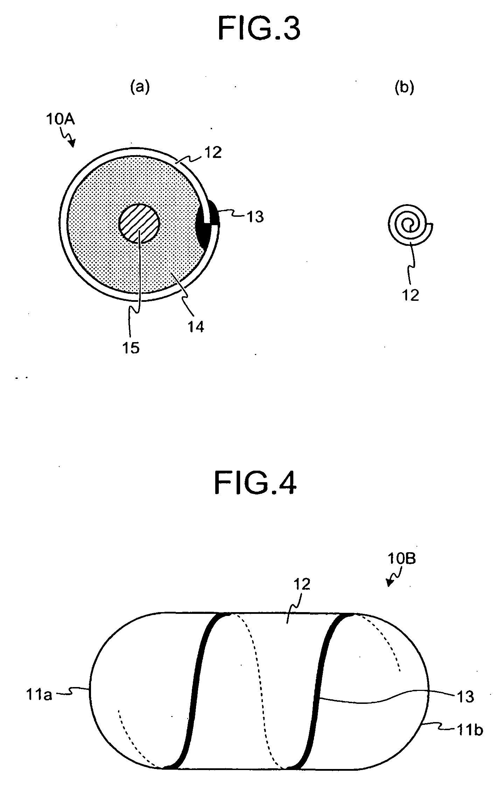

FIG. 3 is a schematic sectional side view of a lumen passability checking capsule 10A of Modification 1. The insoluble portion 12 of the lumen passability checking capsule 10A of Modification 1, which forms the surface layer of the domed-capsule body, is elastic. As shown in FIG. 3(a), both ends of the missing portion of the insoluble portion 12 does not overlap with each other and are at different levels in the radial direction, and the missing portion is filled with the soluble portion 13. The lumen passability checking capsule 10A of Modification 1, which is formed as shown in FIG. 3(a) based on the case shown in FIG. 2, resides in the small intestine. The soluble portion 13, which is linearly, dissolves and the second soluble portion 14 inside dissolves and flows into the outside, so that the lumen passability checking capsule 10A becomes hollow. Thereafter, the lumen passability checking capsule 10A, which maintains the domed capsule shape by the presence of the soluble portion 13, deforms and curls up in a direction such that the diameter is made smaller due to the elasticity of the insoluble portion 12 as shown in FIG. 3(b). Thus, the lumen passability checking capsule 10A easily passes through a constricted portion.

### (Modification 2)

FIG. 4 is a schematic perspective view of a lumen passability checking capsule 10B of Modification 2. The soluble portion 13 of the lumen passability checking capsule 10B of Modification 2 is provided to the insoluble portion 12 linearly and spirally in the circumferential direction on the longitudinal axis of the domed-capsule body. When the lumen passability checking capsule 10B of Modification 2 resides in the small intestine, the soluble portion 13, which is spiral, dissolves, and the second soluble portion 14 dissolves and flows into the outside, the lumen passability checking capsule 10B becomes hollow. Thereafter, the rigidity of the insoluble portion 12 of the lumen passability checking capsule 10B, which maintains the domed-capsule shape by the presence of the soluble portion 13, lowers, so that the insoluble portion 12 cannot maintain the domed-capsule shape and crumples in the axial direction into pieces. Thus, the insoluble portion 12 passes through a constricted portion easily. The number of lines that forms the spiral can be appropriately set. It is preferable that the ends of the spiral be formed such that they extend to the ends of the convex portions 11a and 11b as much as possible.

### (Modification 3)

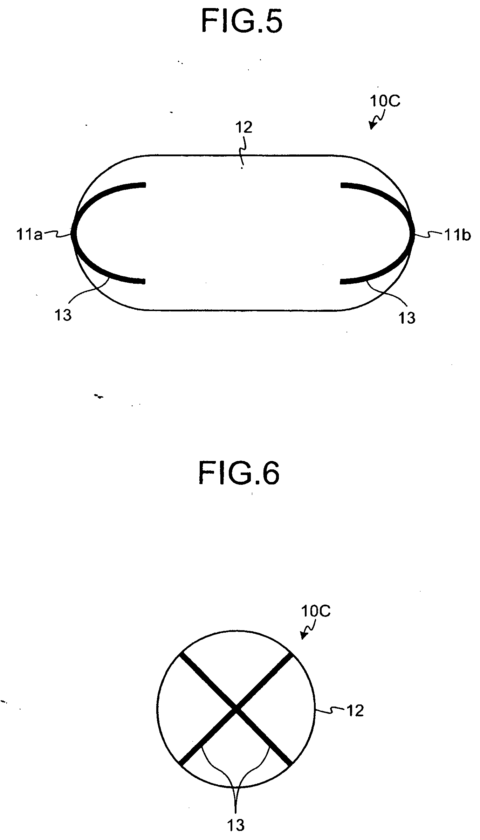

FIG. 5 is a schematic perspective view of a lumen passability checking capsule 10C of Modification 3, and FIG. 6 is an end surface side view of the lumen passability checking capsule 10C. The soluble portion 13 of the lumen passability checking capsule 10C of Modification 3 is provided in lines that intersect in an X shape to the insoluble portion 12 in each of the convex portions 11a and 11b at both ends of the domed-capsule body. When the lumen passability checking capsule 10C of Modification 3 resides in the small intestine and the soluble portion 13 in the X shape dissolves, the second soluble portion 14 dissolves and flows into the outside, so that the lumen passability checking capsule 10C becomes hollow. Thereafter, because the high rigidity of the convex portions 11a and 11b on both ends decreases, the insoluble portion 12, which maintains the domed-capsule shape by the presence of the soluble portion 13, cannot maintain the domed-capsule shape and the insoluble portion 12 becomes cylindrical, i.e., hollow, and easily collapses. Thus, the insoluble portion 12 passes through a constricted portion easily. The number of lines that intersect in the convex portions 11a and 11b is not limited to two. The number may be increased such that the insoluble portion 12 collapses more easily. The soluble portion 13 that is linearly formed in each of the convex portions 11a and 11b may be formed such that it extends toward the center in the longitudinal direction to make the insoluble portion 12 collapse more easily.

### Second example

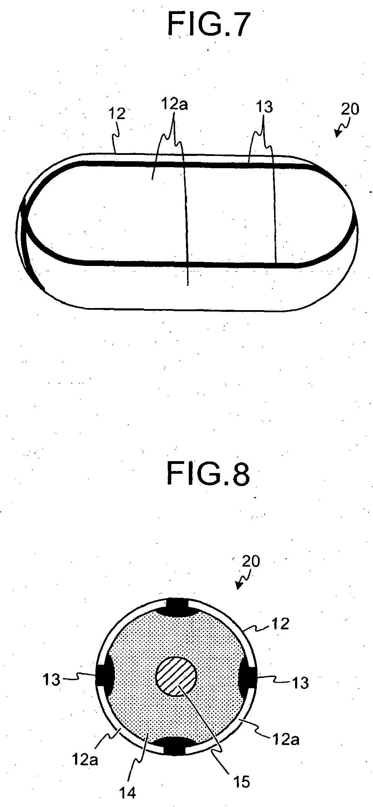

A lumen passability checking capsule according to a second example useful for understanding the present invention is explained below with reference to FIGs. 7 and 8. FIG. 7 is a schematic perspective view of a configuration example of a lumen passability checking capsule 20 according to the second example, and FIG. 8 is a center sectional side view of the lumen passability checking capsule 20.

The lumen passability checking capsule 20 according to the second example is configured based on the lumen passability checking capsule 10 according to the first example, basically. The soluble portions 13 is formed on the insoluble portion 12 in two lines in circles in their directions, so that the insoluble portion 12 is divided, i.e., the insoluble portion 12 includes a plurality of, for example, four, sheet members 12a that are connected with each other with the soluble portion 13. In other words, the soluble portions 13 has a structure of a combination of the first example and Modification 3 in which the portions that are linearly formed in the longitudinal direction and the portions formed linearly in the convex portions 11a and 11b continuously divide the insoluble portion 12 in the longitudinal direction, and in which the domed-capsule shape can be maintained by the presence of the soluble portions 13.

When the lumen passability checking capsule 20 according to the second example resides in the small intestine and the soluble portions 13 in lines dissolve, the insoluble portion 12 of the lumen passability checking capsule 20, which maintains the domed-capsule shape by the presence of the soluble portions 13, cannot maintain the domed-capsule shape and deforms in a direction such that the insoluble portion 12 is definitely divided into the sheet members 12a and becomes smaller. Thus, the lumen passability checking capsule 20 passes through, for example, a constricted portion easily. The number of lines of the soluble portion 13 is not limited to two. The number may be increased such that the number of sheet members of the insoluble portion 12 is increased and each sheet member becomes smaller.

### (Modification 4)

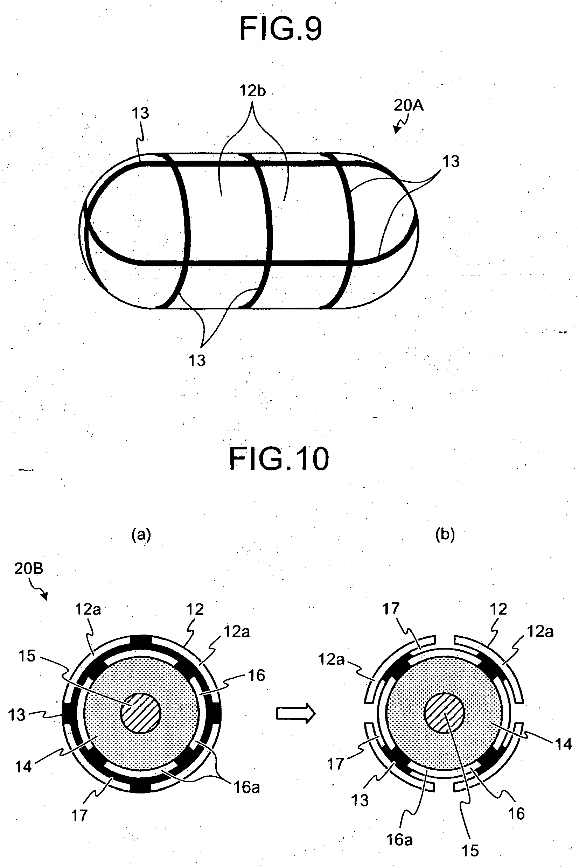

FIG. 9 is a schematic perspective view of a lumen passability checking capsule 20A of Modification 4. The soluble portion 13 of the lumen passability checking capsule 20A of Modification 4 is provided linearly not only in circles in the longitudinal direction but also in circles in the circumferential direction on the longitudinal direction, so that the insoluble portion 12 is divided into a plurality of further segmental sheet members 12b. In the lumen passability checking capsule 20A of Modification 4, when the soluble portion 13 dissolves, the insoluble portion 12 is segmented into the sheet members 12b, so that the insoluble portion 12 passes through a constricted portion more easily. The number of lines of the soluble portion 13 may be appropriately set. In addition, the longitudinal direction and the circumferential direction may be oblique, or can be combined with a spiral shape.

### (Modification 5)

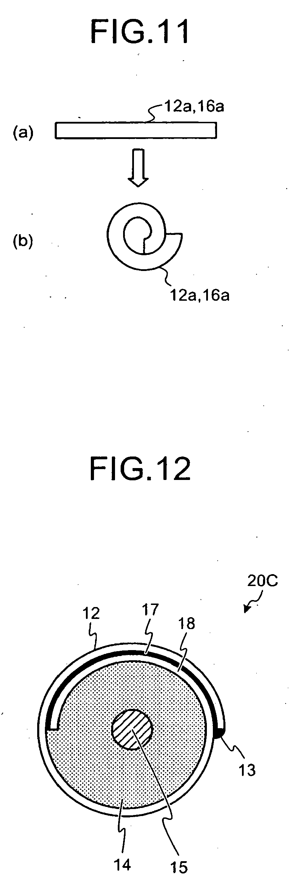

FIG. 10 is a center sectional side view of a lumen passability checking capsule 20B of Modification 5, and FIG. 11 is a cross-sectional view representing how a divided insoluble portion deforms. As shown in FIG. 10(a) a second insoluble portion 16 that is made of a material insoluble in the body is formed in a layer in the insoluble portion 12 approximately in parallel through the soluble portion 13, so that the two-layered insoluble portion is achieved. In addition, as in the case shown in FIG. 7, a missing portion that divides the second insoluble portion 16 linearly along the longitudinal direction into segments of a plurality of sheet members 16a is formed in a part of the second insoluble portion 16, and the missing portion is filled with the soluble portion 13 to connect the sheet members 16a. The second insoluble portion 16 divides the soluble portion (the soluble portion 13 and the second soluble portion 14), which forms, in the soluble portions 13 and 14a, a dissolution route 17 that progresses from a part (missing portion) of the surface to the inside in the form of a non-continuous arc in the cross sectional along the circumferential direction. The dividing position (missing portion) of the insoluble portion by the soluble portion 13 and the dividing position (missing portion) of the second insoluble portion 16 by the soluble portions 13 are not aligned in the circumferential direction on the longitudinal axis of the domed-capsule body. In Modification 5, they are not aligned with equal intervals such that the dissolution route 17 is set longer equally as much as possible. The sheet members 12a and 16b that constitute the insoluble portions 12 and 16 are formed of elastic members that have a characteristic that they curl by elasticity.

When the lumen passability checking capsule 20B of Modification 5 resides in the small intestine, the soluble portion 13 on the surface layer, which is linearly, dissolves from the surface to the inside along the dissolution route 17 whose arc shape in the cross section and that is formed by the second insoluble portion 16 as shown in FIG. 10(b). After the soluble portion 13 between the insoluble portion 12 and the second insoluble portion 16 dissolves completely, the insoluble portion 12 of the lumen passability checking capsule 20B, which maintains the outer shape of the domed-capsule body by the presence of the soluble portion 13, cannot maintain the domed-capsule shape and the insoluble portion 12 is definitely divided into the sheet members 12a. Thereafter, the solving shifts to the soluble portion 13 filled into the missing portion of the second insoluble portion 16. When the soluble portion 13 of this portion dissolves, the second insoluble portion 16 is divided into the sheet members 16a.

Because the sheet members 12a and 16a divided are made of elastic members having a characteristic that they curl by elasticity, they naturally curl and becomes more smaller as shown in FIG. 11(b) from the flat sheet state shown in FIG. 11(a), so that they easily pass through, for example, a constricted portion. In the case of the lumen passability checking capsule 20B of Modification 5, the dissolution route 17 is formed with the second insoluble portion 16 such that the dissolution of the soluble portion 13 takes time. This structurally extends the time taken by the soluble portion 13 to dissolve to, for example, three days or more, so that the domed-capsule shape can be maintained for an extended time.

### (Modification 6)

FIG. 12 is a center sectional side view of a lumen passability checking capsule 20C of Modification 6. The insoluble portion 12 of the lumen passability checking capsule 20C of Modification 6 is spirally formed in a layer approximately in parallel such that a part of the insoluble portion 12, e.g., a half circumference of the insoluble portion 12, overlaps through the soluble portion 13, which is sheet-shaped. The exposed portion of the soluble portion 13 on the surface layer is provided linearly along the longitudinal direction of the domed-capsule body. In addition, a portion of the insoluble portion 12 existing inside constitutes a second insoluble portion 18 that divides the soluble portion (between the second soluble portion 14 and the soluble portion 13) to form the dissolution route 17 by the soluble portion 13 along the circumferential direction in an arc in the cross section.

When the lumen passability checking capsule 20C of Modification 6 resides in the small intestine, the dissolution of the soluble portion 13 starts from the linearly portion exposed to the outside on the surface layer, and the dissolution progresses to the inside along the dissolution route 17 in an arc in the cross section. After the soluble portion 13 in the dissolution route 17 dissolves completely, the second soluble portion 14 dissolves in a short time and the lumen passability checking capsule 20C becomes hollow. In addition, the insoluble portion 12 of the lumen passability checking capsule 20C, which maintains the domed-capsule shape by the presence of the soluble portion 13, cannot maintain the domed-capsule shape and, for example, deforms in a direction such that it curls and becomes smaller. Therefore, the lumen passability checking capsule 20C easily passes through, for example, a constricted portion. In the case of the lumen passability checking capsule 20C of Modification 6, the dissolution route 17 is formed by the second insoluble portion 16 such that the dissolution of the soluble portion 13 takes time. This structurally extends the time taken by the soluble portion 13 to dissolve, which maintains the domed-capsule shape for an extended time.

### (Third example)

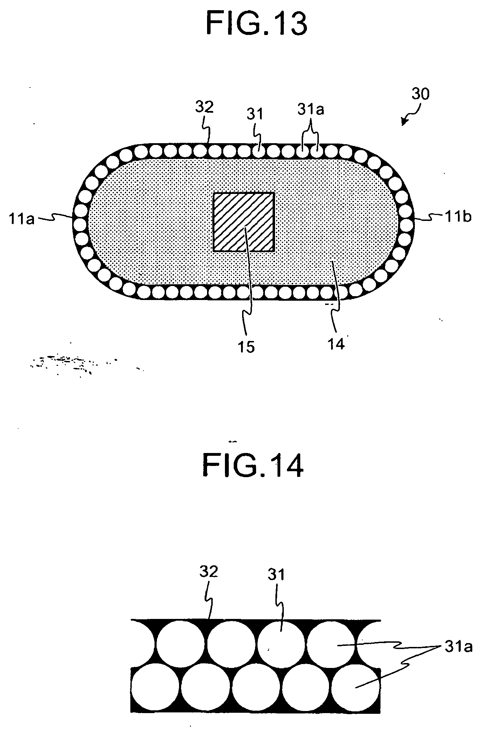

A lumen passability checking capsule according to a third example useful for understanding the present invention is explained below with reference to FIG. 13. FIG. 13 is a center sectional front view of a configuration example of a lumen passability checking capsule 30 according to the third example. An insoluble portion 31 made of a material insoluble in the body includes a large number of the microspheres 31a. The microspheres 31a are connected like a necklace in a thin layer with a soluble portion 32 made of a material soluble in the body, thereby forming the surface layer of the domed-capsule shape. Thus, the soluble portion 32 is present linearly in every direction in the longitudinal direction of the domed-capsule shape, circumferential direction on the longitudinal axis, and in the convex portions 11a and 11b along the sequence of the insoluble portion 31. The material of the insoluble portion 31 and the soluble portion 32 may be same as that in the case explained for the first and second examples. The second soluble portion 14 and the identified portion 15 are appropriately incorporated in the insoluble portion 31.

When the lumen passability checking capsule 30 of the third example resides in the small intestine, dissolution starts from the soluble portion 32 exposed on the surface layer. Because the soluble portion 32 is partly provided such that it fills into the space between the microspheres 31a, the surface of the soluble portion 32 that contacts with body fluids is small and the dissolution takes time, which structurally maintains the domed-capsule shape for an extended time. Even if there is a difference between the progress in dissolution of the soluble portion 32 around the microspheres 31a and the microspheres 31a are partly separated and drop off the surface layer, the domed-capsule shape of the rest of the microspheres 31a that are connected with the soluble portion 32 does not collapse. Therefore, although concave or convex portions are partly caused, the domed-capsule shape can be structurally maintained for an extended time. Thereafter, when the soluble portion 32 mostly dissolves, the insoluble portion 31, which maintains the domed-capsule shape by the soluble portion 32, loses the connecting state between the microspheres 31a. Thus, the insoluble portion 31 is separated into the microspheres 31a and deforms in a direction such that it becomes smaller. The microspheres 31a separated can easily pass through even a constricted portion. If the microspheres 31a are contained in faces discharged by the subject, they serve as an index representing that the lumen passability checking capsule 30 resides in the lumen for a predetermined time or more, so that confirmation about residing can be omitted.

### (Modification 7)

FIG. 14 is a partial cross-sectional view of a lumen passability checking capsule 30A of Modification 7. In the lumen passability checking capsule 30A of Modification 7, a large number of the microspheres 31a that constitute the insoluble portion 31 are provided in a plurality of layers, for example, two layers, through the soluble portion 32 for connecting the microspheres 31a. When the lumen passability checking capsule 30A of Modification 7 resides in the small intestine and the dissolution of the soluble portion 32 progresses, even if the soluble portion 32 serving as the surface layer dissolves, the connecting state of the microspheres 31a of the insoluble portion 31 are connected with each other is maintained by the soluble portion 32 of the lower layer. This structurally maintains the domed-capsule shape for an extended time. After the soluble portion 32 of the lower layer dissolves, the microspheres 31a of the surface layer are separated, which results in a smaller domed-capsule shape. Furthermore, when the rest of the soluble portion 32 dissolves, the microspheres 31a of the lower layer are separated, and the lower layer deforms in a direction such that it becomes smaller. Although the two layers are shown as an example in FIG. 14, the number of layers, the size of the microspheres 31a in each layer, and the ratio of the microspheres 31a to the soluble portion 32 can be appropriately changed depending on a desired dissolution time.

### (Fourth example)

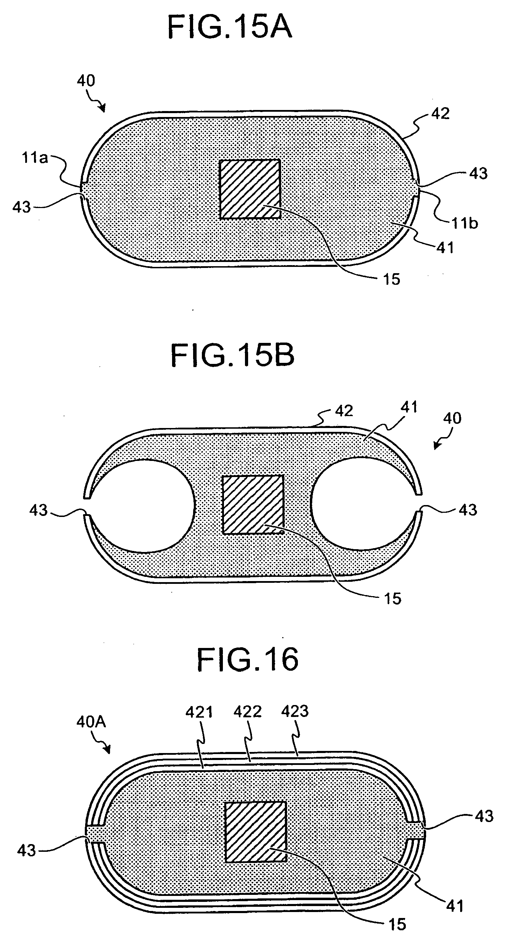

A lumen passability checking capsule according to a fourth example useful for understanding the present invention is explained below with reference to FIG. 15. FIG. 15 is a sectional front view of a lumen passability checking capsule 40 according to the fourth example. The lumen passability checking capsule 40 according to the fourth example includes a soluble portion 41, an insoluble portion 42, and an identified portion 15.

The soluble portion 41 is made of a material that dissolves in the body, and has a structure having a domed-capsule shape. The enteric material same as that used in the above-described examples is used for the material that constitutes the soluble portion 41. The insoluble portion 42 has a characteristic in which it is fragile, has no mechanical strength, and cannot maintain the shape by itself. The insoluble portion 42 is made of a material such as a metal film of, for example, gold, that is basically insoluble in the body, and is formed as a film that covers the surface of the soluble portion such that a part of the soluble portion 41, which forms the structure having the capsule shape, is exposed on the surface through an opening 43. The openings 43 is formed in, for example, a center portion of each of the convex portions 11a and 11b on both ends in the longitudinal direction. The insoluble portion 42 is provided in the state where it is fixed to the soluble portion 41. Thus, the insoluble portion 42 can maintain the capsule shape.

When the lumen passability checking capsule 40 having the above configuration resides in the small intestine, dissolution of the soluble portion 41 gradually progresses from the portion exposed to the outside through the opening 43 to the inside as shown in FIG. 15(b). During this period, the insoluble portion 42 serving as the surface layer maintains the domed-capsule shape integrally with the soluble portion 41. Thereafter, after a predetermined time elapses and most of the soluble portion 41 inside dissolves and the lumen passability checking capsule 40 becomes hollow, the soluble portion 41 cannot maintain the domed-capsule shape and collapses. Because the insoluble portion 42, which is a film, cannot maintain the domed-capsule shape by itself, the insoluble portion 42 collapses into pieces at the same time the soluble portion 41 collapses. Therefore, the insoluble portion 42 can pass through even, for example, a constricted portion.

A method of manufacturing the lumen passability checking capsule 40 according to the fourth example is explained below. In a first example manufacturing is achieved by first performing a step of forming the soluble portion 41 soluble in the body as a structure having a domed-capsule shape, and then performing a step of forming a film of the insoluble portion 42 insoluble in the body, excluding a part (the opening 43) of the structure of the soluble portion 41. The film of the insoluble portion 42 is fixed to the surface of the soluble portion 41.

In a second example, manufacturing is achieved by first performing a step of forming the soluble portion 41 soluble in the body as a structure having a domed-capsule shape, then performing a step of forming and fixing a film of the insoluble portion 42 insoluble in the body on the entire surface of the structure of the soluble portion 41, and performing a step of removing a part (the opening 43) of the film of the insoluble portion 42.

The step of forming a film of the insoluble portion 42 on the surface of the structure of the soluble portion 41 may be a method of depositing a material constituting the insoluble portion 42, or a method of spraying it and then hardening it. Alternatively, a method using a liquid material may be used, in which the structure of the soluble portion 41 is soaked in the liquid material and then it is hardened. The step of forming a film of the insoluble portion 42, excluding a part of the structure of the soluble portion 41, in the first example may be achieved by masking the surface of the structure of the soluble portion 41, forming a film on the surface, and removing the masked portion to form a thin film of the insoluble portion 42 on only the portion not masked. The identified portion 15 may be metal or barium that is radiopaque. Alternatively, the insoluble portion 42 or the soluble portion 41 may be radiopaque and serve as the identified portion 15. In this case, the structure is simplified, the manufacturing is easier, and the dissolution state can be confirmed with x-rays.

### (Modification 8)

FIG. 16 is sectional front view of a lumen passability checking capsule 40A of Modification 8. In the lumen passability checking capsule 40A of Modification 8, films of the insoluble portion 42 are formed in multiple layers, for example, three layers, as indicated by the reference numerals 42a, 42b, and 42c, on the surface of the structure of the soluble portion 41. In the lumen passability checking capsule 40A of modification 8, even if the surface of the lumen passability checking capsule 40a of modification 8 is damaged before deglutition or in the lumen, the soluble portion 41 inside is prevented from being exposed by the multi-structured insoluble portions 42a, 42b, and 42c. Thus, the dissolution of the soluble portion 41 does not progress from parts other than the opening 43, which prevents the time during which the domed-capsule shape cannot be maintained from being shortened.

The lumen passability checking capsule 40A of Modification 8 is manufactured by, first, performing a step of forming the soluble portion 41 soluble in the body as a structure having a domed-capsule shape, and repeatedly performing, for necessary times, a step of forming and fixing a film of the insoluble portion 42 insoluble in the body, excluding a part (the opening 43) of the structure of the soluble portion 41. When performing the step of forming the soluble portion 41 as a structure having a domed-capsule shape, by forming the soluble portion 41 in a size smaller than the size of an objective domed-capsule shape, the size of the lumen passability checking capsule 40A that is finally manufactured can be adjusted to the size of the objective domed-capsule shape. In addition, the insoluble portion 42 is formed on the surface of the soluble portion 41 such that the soluble portion 41 and the insoluble portion 42 are fixed to each other, and the insoluble portion 42 is formed on the surface of the insoluble portion 42 such that the insoluble portions 42 are fixed to each other.

In the case of the lumen passability checking capsules 40 and 40A according to the fourth embodiment and Modification 8, the soluble portion 41 forms the structure having the domed-capsule shape. Alternatively, the case can be applied as well where the insoluble portion 42 is provided, for example, in a thin layer such that it is flexible. In this case, after the soluble portion 41 dissolves, the insoluble portion 42 crumples and thus becomes smaller.

### (First Embodiment)

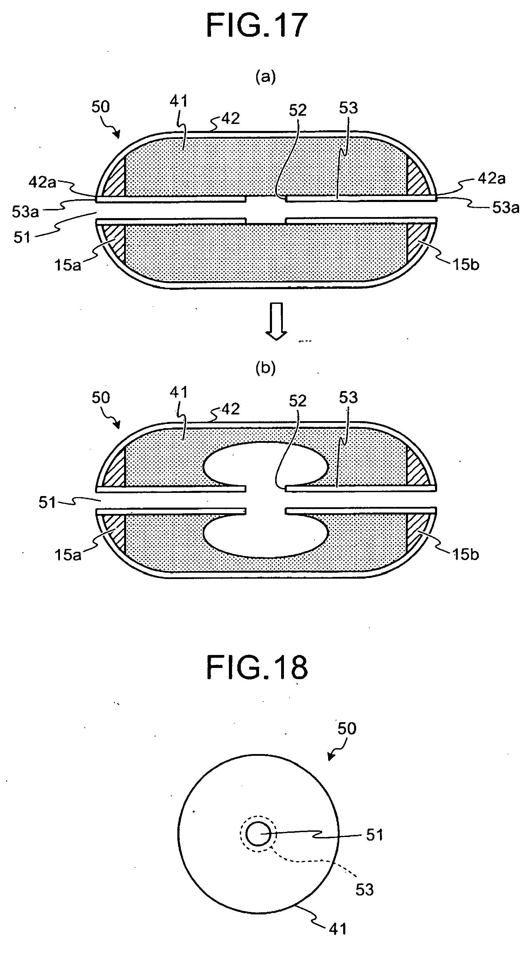

A lumen passability checking capsule according to a first embodiment of the present invention is explained below with reference to FIGs. 17 and 18. FIG. 17 is a sectional front view of a configuration example of a lumen passability checking capsule 50 according to the first embodiment, and FIG. 18 is a side view of the lumen passability checking capsule 50.

In the lumen passability checking capsule 50 according to the first embodiment, a through hole 51 positioned on the axial center of the soluble portion 41, which forms the structure having the domed-capsule shape, and that penetrates in the longitudinal direction is formed, and a second insoluble portion 53 provided on the inner surface of the soluble portion 41, excluding a part of the through hole 51, specifically, an opening 52 at the center in the longitudinal direction in the first embodiment. An end 53a in the longitudinal direction of the second insoluble portion 53 that is made of a material insoluble in the body is integrally connected to an end 42a of the insoluble portion 42 on the surface of the domed-capsule shape. The material of the second insoluble portion 53 may be same as or different from that of the insoluble portion 42. The identified portion 15 that is incorporated in the lumen passability checking capsule 50 are divided into identified portions 15a and 15b in the soluble portion 41 and the identified portions 15a and 15b are positioned separately at the most inward portions on both ends in the longitudinal direction that are the remotest from the openings 52. The insoluble portions 42 and 53 have characteristics that they are fragile have no mechanical strength. The insoluble portions 42 and 53 are formed such that they are fixed to the soluble portion 41.

When the lumen passability checking capsule 50 having the above configuration resides in the small intestine, dissolution gradually progresses from a part of the soluble portion 41 exposed to the outside through the opening 52 at a center portion of the through hole 51 toward the inside as shown in FIG. 17(b). During this period, the insoluble portion 42 serving as the surface layer maintains the domed-capsule shape by the soluble portion 41 inside. After a predetermined time elapses, most of the soluble portion 41 inside dissolves, and the lumen passability checking capsule 50 becomes hollow, the soluble portion 41 cannot maintain the domed-capsule shape and collapses into pieces. Because the insoluble portion 42 that is a film cannot maintain the domed-capsule shape by itself, the insoluble portion 42 collapses into pieces at approximately the same time when the soluble portion 41 collapses. Therefore, the insoluble portion 42 can pass through even, for example, a constricted portion. Due to the collapse of the soluble portion 41 and the insoluble portions 42 and 53, the identified portions 15a and 15b are separated into pieces, pass through a constricted portion, and are discharged.

In the lumen passability checking capsule 50 according to the first embodiment, dissolution of the soluble portion 41 progresses from the openings 52, which are formed at the center portion, through the through hole 51 as a part irrelevant to maintaining of the domed-capsule shape. Therefore, the capsule shape can be structurally maintained for an extended time. In addition, because the ends 42a of the insoluble portion 42 are connected to the ends 53a of the insoluble portion 53 and are continuously protected, collapse of the capsule shape due to dissolution of the soluble portion 41 from near the ends 42a of the insoluble portion 42 is not caused, which also makes it possible to structurally maintain the capsule shape for an extended time.

In the lumen passability checking capsule 50 according to the first embodiment, the identified portion 15 is divided into the identified portion 15a and 15b and the identified portion 15a and 15b are separately arranged. Therefore, the capsule state can be confirmed by confirming whether there are the identified portions 15a and 15b after the predetermined time elapses, using an identifying device. When the identified portions 15a and 15b that are recognized disperse, it can be determined that the lumen passability checking capsule 50 collapses in the lumen. In contrast, when the identified portions 15a and 15b that are recognized are in the original state where they are separately arranged, it can be determined that the lumen passability checking capsule 50 does not collapse and resides in the lumen. Specifically, because the identified portions 15a and 15b are arranged in the remotest portions from the opening 52, the positional relationship between the identified portions 15a and 15b can be maintained until the lumen-passability checking capsule 50 finally collapses, and it can be appropriately determined whether the lumen passability checking capsule 50 collapses in the lumen. The identified portion may be a radiopaque metal and insoluble in the intestines or barium that is soluble in the intestines. The soluble portion 41 and the insoluble portions 42 and 53 may be radiopaque and serve as the identified portion 15 as in the case of the fourth example.

In the case of the lumen passability checking capsule 50 according to the first embodiment, the soluble portion 41 forms the structure having the domed-capsule shape. The case can be similarly applied where the insoluble portion 42 is provided, for example, in a thin layer to be flexible such that it forms a structure having a domed-capsule shape. In this case, after the soluble portion 41 dissolves, the insoluble portion 42 crumples and thus becomes smaller.

### (Fifth example)

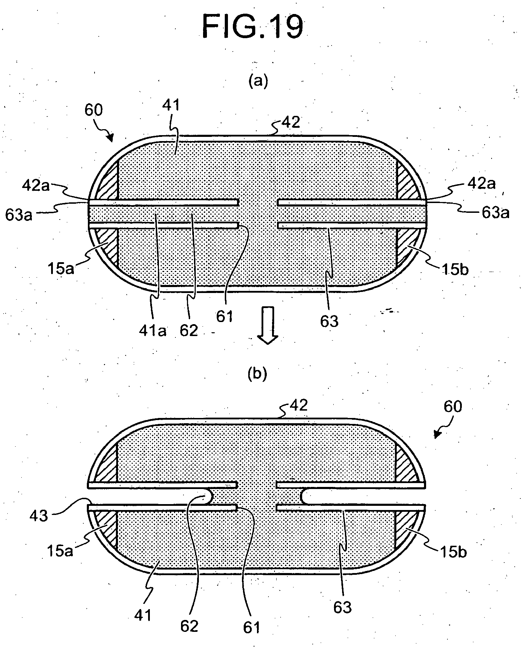

A lumen passability checking capsule according to a fifth example useful for understanding the present invention is explained below with reference to FIG. 19. FIG. 19 is a sectional front view of a configuration example of a lumen passability checking capsule 60 according to the fifth example.

The lumen passability checking capsule 60 according to the fifth example includes a second insoluble portion 63 that is provided to the soluble portion 41 such that it extends in a longitudinal center direction, which forms the structure having the domed-capsule shape, and that divides the soluble portion 41 cylindrically along the longitudinal direction, thereby forming, in the soluble portion 41, a dissolution route 62 that progresses from the opening 43 outside that is a part of the surface to the inside through an opening 61 at the inner center. Ends 63a of the second insoluble portion 63 made of a material insoluble in the body are integrally connected with the ends 42a of the insoluble portion 42 on the surface of the domed-capsule shape. The material of the second insoluble portion 63 may be same as or different from that of the insoluble portion 42. The identified portion 15 incorporated in the lumen passability checking capsule 60 is divided into the identified portions 15a and 15b, and the identified portions 15a and 15b are separately arranged in the soluble portion 41 at both of the most inward portions of the dissolution route 62. The insoluble portions 42 and 63 have characteristics that they are fragile and have no mechanical strength. The insoluble portions 42 and 63 are formed such that they are fixed to the soluble portion 41.

In the lumen passability checking capsule 60 according to the fifth example is manufactured by providing the second insoluble portion 63 on the surface of a soluble portion 41a, which is formed cylindrically, excluding the portions of the openings 43 and 61, and providing the soluble portion 41b on the surface in a desired domed-capsule shape to form a structure, and forming and fixing a film of the insoluble portion 42 on the surface of the structure.

When the lumen passability checking capsule 60 having the above configuration resides in the small intestine, dissolution starts from the portions of the soluble portion 41 exposed to the outside through the openings 43, progresses to the inside of the second insoluble portion 63 along the dissolution route 62 as shown in FIG. 19(b), and progresses to the outside of the second insoluble portion 63 through the openings 61. During this period, the insoluble portion 42 serving as the surface layer maintains the domed-capsule shape by the soluble portion 41 inside. Thereafter, when a predetermined time elapses and most of the soluble portion 41 inside dissolves and the lumen passability checking capsule 60 becomes hollow, the soluble portion 41 cannot maintain the domed-capsule shape and collapses. Because the insoluble portion 42, which is a film, cannot maintain the domed-capsule shape by itself, the insoluble portion 42 collapses at approximately the same time the soluble portion 41 collapses. Therefore, the insoluble portion 42 can pass through even, for example, a constricted portion. Due to the collapse of the soluble portion 41 and the insoluble portions 42 and 63, the identified portions 15a and 15b disperse as well, pass through the constricted portion, and are discharged.

In the lumen passability checking capsule 60 according to the fifth example, the dissolution route 62 that progresses from a part of the surface to the inside is formed in the soluble portion 41 by dividing the soluble portion 41 with the second insoluble portion 63. Forming the dissolution route 62 longer makes it possible to structurally extend the time taken by the soluble portion 41 to dissolve in the lumen to, for example, three days or more, which maintains the capsule shape for an extended time. Specifically, because the second insoluble portion 63 that forms the dissolution route 62 is provided such that it extends toward the center of the domed-capsule shape, the dissolution route 62 in which the soluble portion 41 dissolves can be formed from a center portion of the domed-capsule shape toward the outside such that portions irrelevant to maintaining of the domed-capsule shape dissolve first. This extends the time until the soluble portion 41 near the insoluble portion 42, which serves as the outer surface of the domed-capsule shape, dissolves. Furthermore, because the ends 42a of the insoluble portion 42 are connected to the ends 63a of the second insoluble portion 63 and they are continuously protected, collapse of the capsule shape due to the dissolution of the soluble portion 41 near the ends 42a of the insoluble portion 42 is not caused, which also makes it possible to structurally maintain the capsule shape for an extended time. Furthermore, because the capsule shape can be maintained for an extended time by the dissolution route 62, materials that are used for the soluble portion 41 can be selected from more choices, which achieve simple manufacturing and lower costs.

In the lumen passability checking capsule 60 according to the fifth example, the identified portion 15 is divided into the identified portions 15a and 15b and the identified portions 15a and 15b are separately arranged as in the case of the first embodiment. Therefore, the capsule state can be confirmed by confirming whether there are the identified portions 15a and 15b, using an identifying device, after the predetermined time elapses. When the identified portions 15a and 15b that are identified disperse, it can be determined that the lumen passability checking capsule 60 collapses in the lumen. In contrast, when the identified portions 15a and 15b that are identified are in the original state where they are separately arranged, it can be determined that the he lumen passability checking capsule 60 does not collapse and resides in the lumen. Specifically, because the identified portions 15a and 15b are arranged in the most inward portions of the dissolution route 62, the positional relationship between the identified portions 15a and 15b can be maintained until the lumen passability checking capsule 60 finally collapses, and it can be appropriately determined whether the lumen passability checking capsule 60 collapses in the lumen. The identified portions may be a metal that is radiopaque and insoluble in the intestines, or barium that is soluble in the intestines. The soluble portion 41 and the insoluble portions 42 and 63 may be radiopaque and serve as the identified portion 15 as in the case of the fourth example.

### (Modification 9)

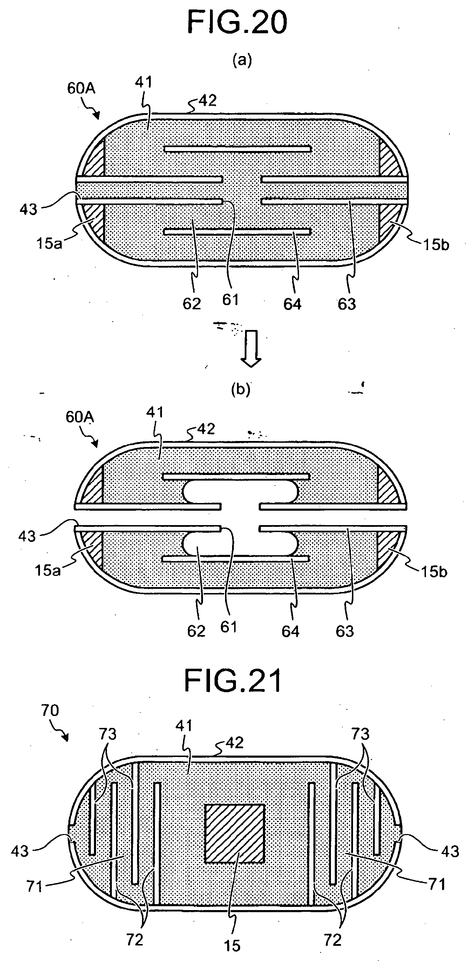

FIG. 20 is a sectional front view of a lumen passability checking capsule 60A of Modification 9. In the lumen passability checking capsule 9 of modification 9, the dissolution route 62 is further divided and compartmentalized by providing, in the soluble portion 41, a cylindrical third insoluble portion 64 made of a material insoluble in the body on an outer side with respect to the second insoluble portion 63, which is cylindrical. The third insoluble portion 64 is provided around a center portion including the area around the opening 61 such that the dissolution route 62 extends in the following order: the opening 43, in the second insoluble portion 63, the opening 61, between the second and third insoluble portions 63 and 64, and between the third insoluble portion 64 and the insoluble portion 42. Because the dissolution route 62 is formed longer by compartmentalization in the lumen passability checking capsule 60A of Modification 9, the time necessary for dissolution of the soluble portion 41 can be extended structurally, which makes it possible to maintain the domed-capsule shape more longer. Because the capsule shape can be maintained by the dissolution route 62 for an extended time, a material used for the soluble portion 41 can be selected from more choices, which achieves simple manufacturing and lower costs.

In the case of the lumen passability checking capsules 60 and 60A according to the fifth example and Modification 9, the soluble portion 41 forms the structure having the domed-capsule shape. Alternatively, the case where the insoluble portion 42 is provided, for example, in a thin layer to be flexible such that it forms a structure having a domed-capsule shape may be similarly applied. In this case, after the soluble portion 41 dissolves, the insoluble portion 42 crumples and thus becomes smaller.

### (Sixth example)

A lumen passability checking capsule according to a sixth example of the present invention is explained below with reference to FIG 21. FIG. 21 is a sectional front view of a configuration example of a lumen passability checking capsule 70 according to the sixth example.

In the lumen passability checking capsule 70 according to the sixth example, a second insoluble portion 72 that divides the soluble portion 41 in the direction perpendicular to the longitudinal axis is provided like teeth of a comb to form a dissolution route 71 that progresses from the opening 43 to an inner center side. Furthermore, a third insoluble portion 73 that further divides the dissolution route 71 in the direction perpendicular to the longitudinal axis from the side opposite to the second insoluble portion 72 to further divide and compartmentalize the dissolution route 71 is provided like teeth of a comb. In other words, the second and third insoluble portions 72 and 73 made of a material insoluble in the body are formed like teeth of a comb that interdigitate, so that the dissolution route 71 that starts from the opening 43 is bent back like a maze to be longer. The insoluble portions 42, 72, and 73 have characteristics that they are fragile and have no mechanical strength, and are formed in the state where they are fixed to the soluble portion 41.

When the lumen passability checking capsule 70 having the above configuration resides in the small intestine, dissolution starts from the portions of the soluble portion 41 exposed to the outside through the openings 43, and progresses in the soluble portion 41 along the dissolution route 71, which is formed like a maze. During this period, the insoluble portion 42 serving as the surface layer maintains the domed-capsule shape by the soluble portion 41 inside. Thereafter, when a predetermined time elapses and most of the soluble portion 41 inside dissolves and the insoluble portion 42 becomes hollow, the soluble portion 41 cannot maintain the domed-capsule shape and collapses. Because the insoluble portion 42, which is a film, cannot maintain the domed-capsule shape by itself, the insoluble portion 42 collapses at approximately the same time the soluble portion 41 collapses into pieces. Therefore, the insoluble portion 42 can pass through even, for example, a constricted portion.

In the lumen passability checking capsule 70 according to the sixth example, the dissolution route 71 is formed by dividing and compartmentalizing the soluble portion 41 with the second and third insoluble portions 72 and 73, so that the dissolution route 71 becomes longer. This structurally extends the time during which the soluble portion 41 dissolves in the lumen, which makes it possible to maintain the capsule shape for an extended period of time. Thus, the capsule shape can be definitely maintained for a predetermined time necessary to confirm patency, and patency of the lumen can be definitely confirmed. The identified portion may be a metal that is radiopaque and insoluble in the intestines, or barium that is soluble in the intestines. The soluble portion 41 and the insoluble portion 42, 72, and 73 may be radiopaque and serve as the identified portion 15 as in the case of the fourth example.

### (Seventh example)

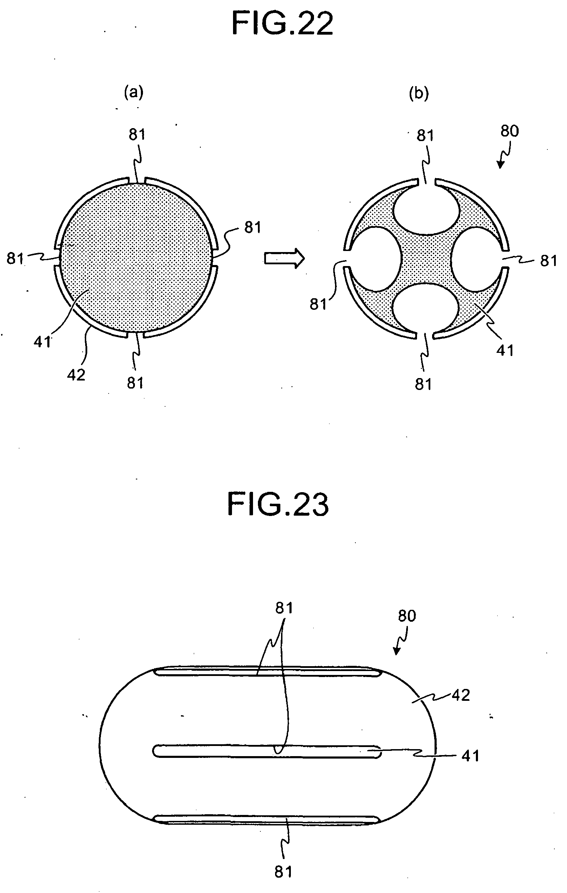

A lumen passability checking capsule according to a seventh example useful for understanding the present invention is explained below with reference to FIGs. 22 and 23. FIG. 22 is a center portion sectional side view of a configuration example of a lumen passability checking capsule 80 according to the seventh example, and FIG. 23 is a front view of the lumen passability checking capsule 80.

In the lumen passability checking capsule 80, openings 81 through which the soluble portion 41 is exposed from the insoluble portion 42 on the surface are formed linearly in the longitudinal direction in a plurality of positions, for example, four positions, on the surface of the soluble portion 41 that forms the structure having the domed-capsule shape.

When the lumen passability checking capsule 80 having the above configuration resides in the small intestine, dissolution of the soluble portion 41 gradually progresses from the portions of the openings 81 exposed on the surface toward the inside as shown in FIG. 22(b). During this period, the insoluble portion 42 serving as the surface layer maintains the domed-capsule shape by the soluble portion 41 inside. Thereafter, when a predetermined time elapses and most of the soluble portion 41 inside dissolves, the insoluble portion 42, which is a film, cannot maintain the domed-capsule shape by itself and collapses into pieces or crumples. Thus, the insoluble portion 42 can pass through even, for example, a constricted portion.

### (Modification 10)

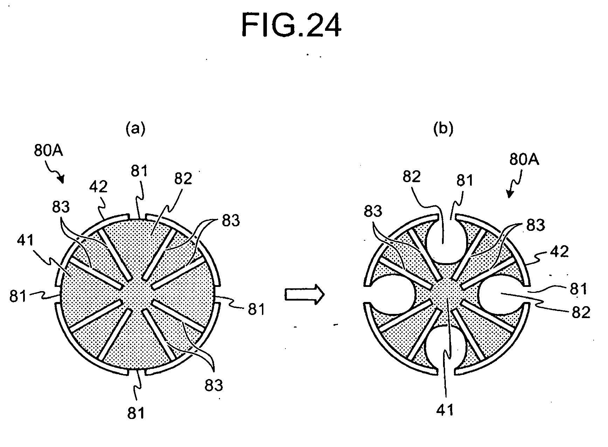

FIG. 24 is a center portion sectional side view of a lumen passability checking capsule 80A of Modification 10. In the lumen passability checking capsule 80A of Modification 10, a second insoluble portion 83 that divides the soluble portion 41 inside in the radial direction to form a dissolution route 82 that progresses from the openings 81 to the inside is radially provided to the inner circumference of the insoluble portion 42 in the soluble portion 41. In other words, the second insoluble portion 83 forms the dissolution route 82 that is bent back radially such that it extends in the following order: the opening 81, the axial center portion, and the inner surface of the insoluble portion 42.

When the lumen passability checking capsule 80A having the above configuration resides in the small intestine, dissolution starts from the portions of the soluble portion 41 exposed to the outside through the openings 81 and progresses toward the inside, which is the axial center portion, along the dissolution route 82 formed by the second insoluble portion 83 as shown in FIG. 24(b). During this period, the insoluble portion 42 serving as the surface layer maintains the domed-capsule shape by the soluble portion 41 inside. Thereafter, when a predetermined time elapses and the soluble portion 41 of the portion on the inner surface of the insoluble portion 42 dissolves, the insoluble portion 42, which is a thin film, cannot maintain the domed-capsule shape by itself, and it collapses into pieces or crumples. Thus, the insoluble portion 42 can pass through even, for example, a constricted portion.

In the lumen passability checking capsule 80A of Modification 10, the dissolution route 82 that structurally extends the dissolution time is formed by dividing the soluble portion 41 by the second insoluble portion 83. Therefore, the time during which the soluble portion 41 dissolves in the lumen can be structurally extended, which maintains the capsule shape for an extended time. Thus, the capsule shape can be definitely maintained for a predetermined time necessary to confirm patency, and the patency of the lumen can be definitely confirmed.

In the case of the lumen passability checking capsule 80 and 80A according to the eighth embodiment and Modification 10, the soluble portion 41 forms the structure having the domed-capsule shape. Alternatively, the case where the insoluble portion 42 is provided in a thin layer to be flexible such that the insoluble portion 42 forms a structure having a domed capsule shape can be similarly applied. In this case, after the soluble portion 41 dissolves, the insoluble portion 42 crumples and thus becomes smaller.

### (Second Embodiment)

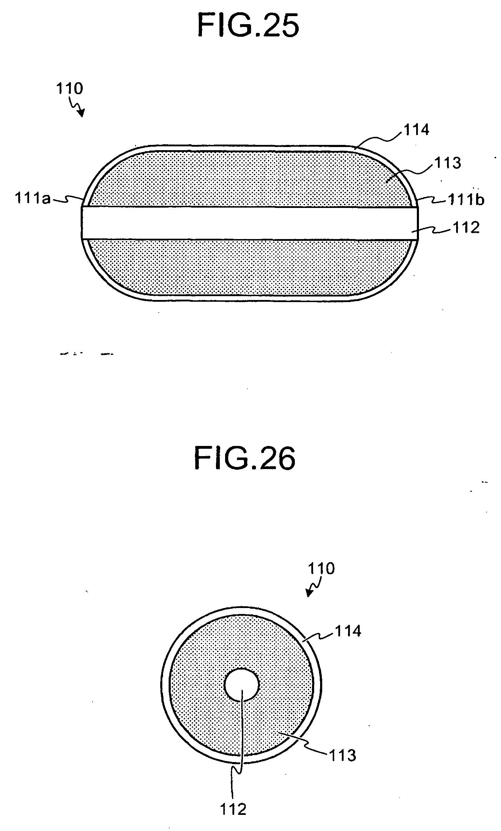

A lumen passability checking capsule according to a second embodiment of the present invention is explained below with reference to FIGs. 25 and 26. FIG. 25 is a sectional front view of a configuration example of a lumen passability checking capsule 110 according to the second embodiment, and FIG. 26 is a center sectional side view of the lumen passability checking capsule 110.

The lumen passability checking capsule 110 according to the second embodiment generally has the same or approximately same shape and size as those of a capsule endoscope for the purpose of internal examination and observation of the lumen of, for example, the intestines and stomach, and can be introduced into a subject of a subject. The lumen passability checking capsule 110 includes a domed-capsule shape having protruding portions 111a and 111b that are semi-sphere and domed on both ends in the longitudinal direction. The lumen passability checking capsule 110, which has such a domed-capsule shape, includes an insoluble portion 114 and a soluble portion 113.

The soluble portion 113 is made of a material that dissolves in and/or erodes with body fluids in the body, and forms a structure having a domed-capsule shape. In the soluble portion 113, a through hole 112 is formed that is positioned at the axial center and that penetrates in the longitudinal direction. An enteric material is used for the material constituting the soluble portion 113. The insoluble portion 114 is made of a material that is basically insoluble in the body, such as a metal film of, for example, gold, and is substantially impermeable (impenetrable) to body fluids. The insoluble portion 114 forms the surface layer of the domed-capsule shape, excluding the openings of the through hole 112.

In the second embodiment, the soluble portion 113 is formed of an enteric material that does not easily dissolve in the stomach but dissolves while it passes through the small intestine and the large intestine. For example, the soluble portion 13 is formed of an alkali-soluble enteric material, such as (acetic acid/succinic acid) hydro propyl methyl cellulose described in Patent Document 3, or a natural polysaccharide-polyalcohol compound discribed in Patent Documents 5 and 6 obtained by uniformly mixing natural polysaccharide, which is selected from carrageenan, alginic acid, alginate, alginate derivative, agar, locust bean gum, guar gum, amylopectin, pectin, xanthan gum, glucomannan, chitin, and pullulan into any one selected from polyalcohol, sugar alcohol, disaccharide, trisaccharide, and oligosaccharide.

The lumen passability checking capsule 110 having the above configuration is introduced into a subject from the mouth a predetermined time (for example, few days) before prior to internal examination and observation of the lumen using a capsule endoscope, which can be used, to confirm beforehand whether the capsule endoscope can pass through the hollow organs of, for example, the small intestine.

The lumen passability checking capsule 110, which is introduced into the body, has the same size and shape as those of the capsule endoscope. If there is an extremely narrow abnormal portion due to, for example, a constricted portion, in the lumen, the lumen passability checking capsule 110 sequentially passes through the lumen of, for example, the stomach, the small intestine, and the large intestine, by peristalsis of the lumen and is discharged to the outside of the body. Because most of the surface layer of the lumen passability checking capsule 110 is formed as the insoluble portion 114, the lumen passability checking capsule 110 moves forward without dissolution with the body fluid, such as gastric juice, before it passes through the stomach. When passing through the small intestine and the large intestine, the lumen passability checking capsule 110 moves forward while the soluble portion 113, which is made of the enteric material, gradually dissolves. During a predetermined time until the soluble portion 113 dissolves, the domed-capsule shape is maintained. If there is no abnormality, such as a constricted portion, in the small intestine, the lumen passage conformation capsule 110 moves forward without residing in the small intestine or the large intestine while maintaining the domed-capsule shape and is discharged to the outside of the body. Thus, it is confirmed that there is no abnormality, such as a constricted portion, in the lumen, i.e., that the capsule endoscope can pass through the lumen smoothly.

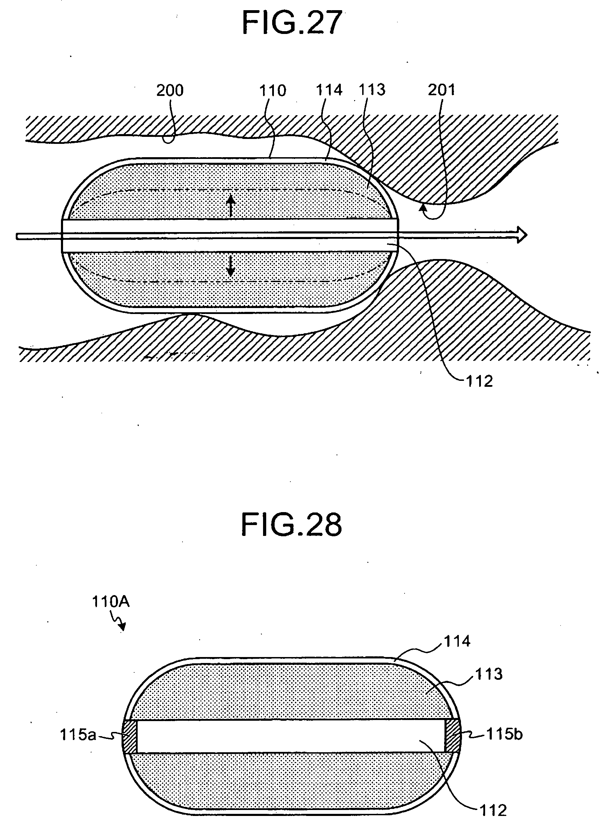

In contrast, if there is an abnormality, such as a constricted portion, in a portion, such as the small intestine, the lumen passability checking capsule 110 is inhibited from moving forward even with peristalsis, and resides in the constricted portion. When the lumen passability checking capsule 110 resides in the small intestine, dissolution of the soluble portion 113 gradually progresses from the portions exposed on the side of the through hole 112 to the inside. During this period, the insoluble portion 114 serving as the surface layer maintains the domed-capsule shape by the soluble portion 113 inside. Thereafter, when a predetermined time elapses, most of the soluble portion 113 dissolves, and the insoluble portion 114 becomes hollow, the insoluble portion 114, which is a film, cannot maintain the domed-capsule shape by itself and collapses into pieces or crumples. Thus, the insoluble portion 114 passes through even a constricted portion 201 of a lumen 200 shown in FIG. 27. Specifically, because the insoluble portion 114 insoluble in the body is formed of a film, the insoluble portion 114, which is left after the soluble portion 113 dissolves, can be definitely made collapse and become smaller, which improves the dischargeability.

When the lumen passability checking capsule 110 is stuck in the constricted portion 201, the soluble portion 113 dissolves as described above the shape is maintained until the domed-capsule shape collapses. However, the lumen passability checking capsule 140 blocks the constricted portion 201 until the soluble portion 113 dissolves and collapses. In the second embodiment, portions of the lumen 200, which are anterior and posterior to the lumen passability checking capsule 110, are always communicated with each other with the through hole 112. Therefore, fluid substances do not reside in the lumen, and for example, acute intestinal obstruction can be prevented from occurring. In addition, the domed-capsule shape can be maintained for a predetermined time.

For example, even when the lumen passability checking capsule dissolves or decomposes after a predetermined time, acute intestinal obstruction may be caused because the lumen passability checking capsule is stuck in the constricted portion in the predetermined time depending on subjects, which requires the lumen passability checking capsule to be taken out by a surgical operation. In addition, it is difficult in many cases to control the predetermined time during which the lumen passability checking capsule dissolves or decomposes, and it may not dissolve or decompose even after much more than the predetermined time, which may cause intestinal obstruction and require a surgical operation. The second embodiment can solve such problems.

It is unnecessary to strictly control the time during which the soluble portion 113 dissolves, i.e., the predetermined time until the domed-capsule shape is lost. The dissolution may complete and the domed-capsule shape may be lost after a relatively long time, for example, one week, because the portions of the lumen, which are anterior and posterior to the lumen passability checking capsule 110, communicate with each other by the through hole 112 and the lumen is never blocked. An enteric material is used only for the outer surface of the soluble portion 113 that contacts with body fluids, and the inside of the soluble portion 113 consists of a material, such as lactose, powder sugar, or powder barium, or a mixture of them that is more soluble than the enteric material.

A method of manufacturing the lumen passability checking capsule 110 according to the second embodiment is explained below. In a first example, manufactured is achieved by first performing a step of forming the soluble portion 113 soluble in the body and the through hole 112 as a structure having a domed-capsule shape, and then performing a step of forming a film of the insoluble portion 114 insoluble in the body, excluding the openings of the through hole 112 formed by the soluble portion 113.

In a second example, the manufacturing is achieved by first performing a step of forming the soluble portion 113 soluble in the body as a structure having a domed-capsule shape, then performing a step of forming a film of the insoluble portion 114 insoluble in the body on the entire surface of the structure formed by the soluble portion 113, and performing a step of forming the through hole 112.

The step of forming a film of the insoluble portion 114 on the surface of the structure formed by the soluble portion 113 may be a method of depositing a material constituting the insoluble portion 114, or a method of spraying it and then hardening it. Alternatively, the step may be a method using a liquid material that serves as the insoluble portion 114, in which the structure formed by the soluble portion 113 is soaked in the liquid material and then hardened. A flexible material having elasticity may be used for the insoluble portion 114.

### (Modification 11)

FIG. 28 is a schematic sectional side view of a lumen passability checking capsule 110A of Modification 11. In the lumen passability checking capsule 110A of Modification 11, soluble portions 115a and 115b that block openings at both ends of the through hole 112 of Modification 9, and that are soluble in the stomach (acid), are further provided. By providing the soluble portions 115a and 115b, the through hole 112 is blocked before they reaches the stomach, so that the lumen passability checking capsule 110A can move smoothly.

### (Modification 12)

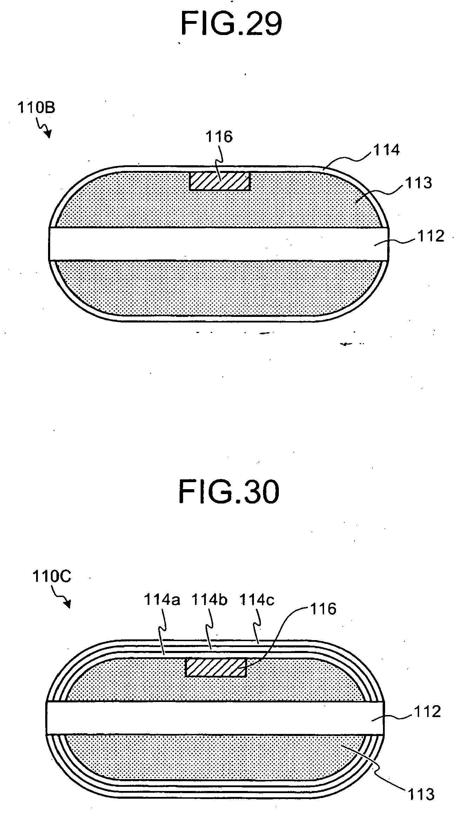

FIG. 29 is a sectional front view of a lumen passability checking capsule 110B of Modification 12. In the lumen passability checking capsule 110B of Modification 12, an identified portion 116 is provided in the soluble portion 113 of the second embodiment in a portion where dissolution starts last.

The identified portion 116 is identified after a predetermined time by an identifying device outside the body of the subject to confirm the positions where the lumen passability checking capsule 110 resides and where it currently passes through. The identified portion 116 is, for example, a radiopaque member, such as barium sulfate, metal (gold, titanium, stainless steel), or an electronic tag, such as an RF-ID (Radio Frequency Identification) tag. If the identified portion 116 is a radiopaque member, it can be confirmed whether the lumen passability checking capsule 110B resides in, for example, a constricted portion by scoping the body of the subject, using an X-ray clairvoyance device. If the identified portion 116 is an electric ID tag, such as an RF-ID tag, electric power is sent to the electric tag, using a tag reader-writer, to operate an IC chip. By receiving necessary information from the tag, the tag can be identified. The identified portion 116 has a size such that it can pass through even a constricted portion. If the insoluble portion 114 serving as the surface layer is metallic, the insoluble portion 114 is used as the identified portion 116, and is not required to be provided in the soluble portion 113. In this case, because the area of the metallic portion is large, identification is easily performed with a commercial metal detector. In Modification 12, not only metallic materials but also resin can be used for the insoluble portion 114.

### (Modification 13)

FIG. 30 is a schematic sectional side view of a lumen passability checking capsule 110C of Modification 13. In the lumen passability checking capsule 110C of Modification 13, films of the insoluble portion 114 are formed in multiple layers, for example, three layers, as indicated by the reference numerals 114a, 114b, and 114c, on the surface of the structure formed by the soluble portion 113. Even if the surface of the lumen passability checking capsule 110C of modification 13 is damaged when the lumen passability checking capsule 110C resides in the small intestine, the soluble portion 113 is prevented from being exposed by the multi-structured insoluble portions 114a, 114b, and 114c. Thus, dissolution of the soluble portion 113 does not progress from portions other than the through hole 112, which prevents the time during which the domed-capsule shape can be maintained from being shortened.

The lumen passability checking capsule 140A of Modification 13 is manufactured by first performing a step of forming the soluble portion 113, which is soluble in the body, as a structure having a domed-capsule shape, and performing a step of forming a film of the insoluble portion 114 insoluble in the body, excluding the openings of the through hole 112, for a required number of times. When performing the step of forming the soluble portion 113 as a structure having a domed-capsule shape, by forming the soluble portion 113 in a size smaller than the size of the objective domed-capsule shape, it is possible to adjust the size of the lumen passability checking capsule 110C finally manufactured to the size of the objective domed-capsule shape.

### (Modification 14)

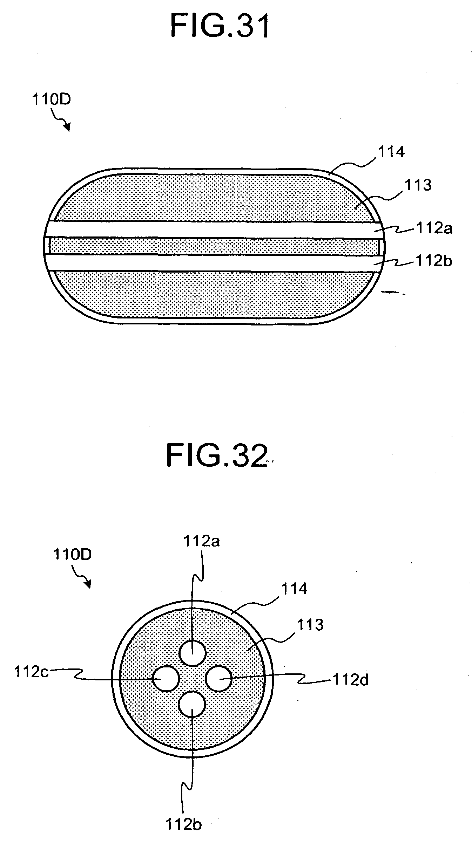

FIG. 31 is a sectional front view of a configuration example of a lumen passability checking capsule 110D of Modification 14. In the lumen passability checking capsule 110D of Modification 14, a plurality of through holes 112a to 112d are provided instead of the through hole 112, which is single. The through holes 112a to 112d each penetrate along the axial center in the longitudinal direction as the through hole 112 does. Therefore, even if one of the through holes is blocked, portions of the lumen, which are anterior and posterior to the lumen passability checking capsule 110D, communicate with each other with other through holes.

### (Third Embodiment)

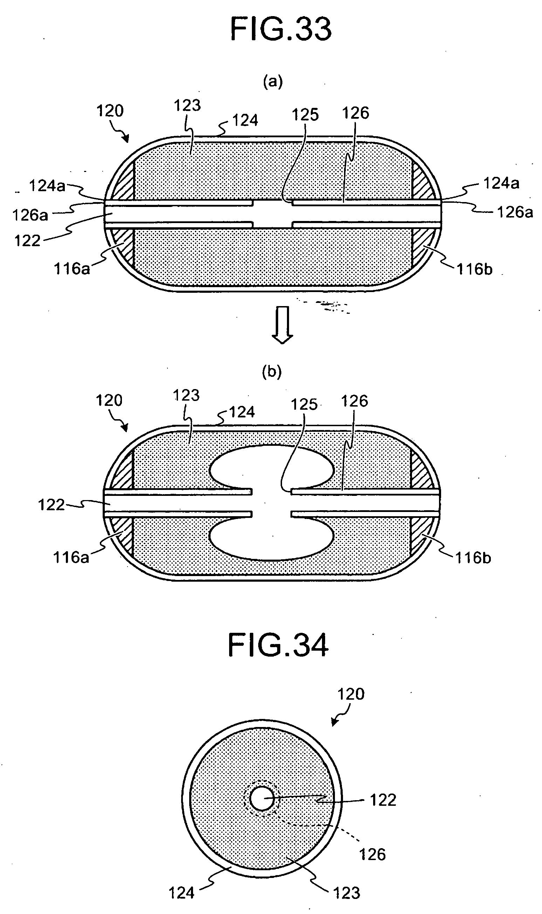

A lumen passability checking capsule according to a third embodiment of the present invention is explained below with reference to FIGs. 33 and 34. FIG. 33 is a sectional front view of a configuration example of a lumen passability checking capsule 120 according to the third embodiment, and FIG. 34 is a center sectional side view of the lumen passability checking capsule 120.

In the lumen passability checking capsule 120 according to the third embodiment, a through hole 122 is formed such that the through hole 122 is positioned on the axial center of a soluble portion 123, which forms a structure having a domed-capsule shape, and penetrates in the longitudinal direction, and an insoluble portion 126 is provided on the inner surface of the soluble portion 123, excluding a part in the through hole 122, specifically, an opening 125 at the center in the longitudinal direction in the third embodiment. Ends 126a of the insoluble portion 126 made of a material insoluble in the body in the longitudinal direction are integrally connected with ends 124a of an insoluble portion 124 on the surface of the domed-capsule shape. The material of the insoluble portion 126 may be same as or different from that of the insoluble portion 124. Identified portions 116a and 16b are divided and separately provided in the soluble portion 123 on the most inward portions on both ends in the longitudinal direction that are the remotest from the opening 125.

When the lumen passability checking capsule 120 having the above configuration resides in the small intestine, dissolution gradually progresses from the portion of the soluble portion 123 that are exposed to the outside through the opening 125 to the inside as shown in FIG. 33(b). During this period, the insoluble portion 124 serving as the surface layer maintains the domed-capsule shape by the soluble portion 123 inside. Thereafter, when a predetermined time elapses, most of the soluble portion 123 inside dissolves, and the insoluble portion 124 becomes hollow, the insoluble portion 114, which is a film, cannot maintain the domed-capsule shape by itself and collapses into pieces or crumples. Thus, the insoluble portion 124 can pass through even a constricted portion. Due to the collapse of the insoluble portion 124, the identified portions 115a and 115b disperse, pass through the constricted portion, and are discharged. Because the through hole 122 is formed, portions of the lumen, which are anterior and posterior to the lumen passability checking capsule, communicate with each other. Thus, even if the lumen passability checking capsule 120 is stuck in, for example, a constricted portion, fluid substances in the lumen can move even in the predetermined time.

In the lumen passability checking capsule 120 according to the third embodiment, the through hole 122 always does not prevent fluid substances from moving through the lumen. Therefore, for example, acute intestinal obstruction can be definitely prevented from occurring in the predetermined time. In addition, it is unnecessary to strictly control the time taken by the soluble portion 123 to dissolve, and a material with a relatively long dissolution time can be selected.

In the lumen passability checking capsule 120 according to the third embodiment, dissolution of the soluble portion 123 progresses from the opening 125, which is formed at the inner center portion through the through hole 122, as a portion irrelevant to maintaining of the domed-capsule shape. Therefore, the capsule shape can be structurally maintained for an extended time. Furthermore, because the ends 124a of the insoluble portion 124 are connected with the ends 126a of the insoluble portion 126 and continuously protected, collapse of the capsule shape due to dissolution of the soluble portion 123 from near the ends 126a of the insoluble portion 126 is not caused, which also structurally maintains the capsule shape for an extended time.

In the lumen passability checking capsule 120 according to the third embodiment, the identified portions 115a and 115b are divided and separately arranged. Therefore, the capsule state can be confirmed by confirming whether there are the identified portions 115a and 115b, using an identifying device, after more than the predetermined time. When the identified portions 115a and 115b that are recognized disperse, it can be determined that the lumen passability checking capsule 120 collapses in the lumen. In contrast, when the identified portions 115a and 115b that are recognized are in the original state where they are separately arranged, it can be determined that the lumen passability checking capsule 120 does not collapse and resides in the lumen. Specifically, because the identified portions 115a and 115b are arranged in the most inward portions that are the remotest from the opening 125, the positional relationship between the identified portions 115a and 115b can be maintained until the lumen passability checking capsule 120 collapses finally, which makes it possible to appropriately determines whether the lumen passability checking capsule 120 collapses in the lumen.

### (Modification 15)

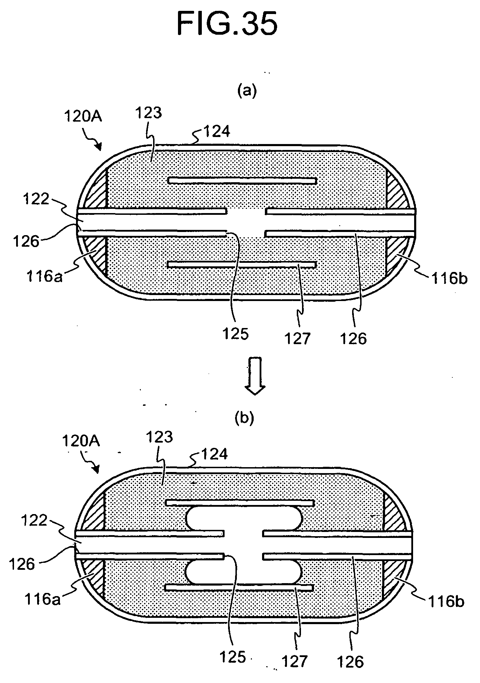

FIG. 35 is a sectional front view of a lumen passability checking capsule 120A of Modification 15. In the lumen passability checking capsule 120A of modification 15, a cylindrical insoluble portion 127 that is made of a material insoluble in the body is provided in the soluble portion 123 on the outer side of an insoluble portion 126, which is cylindrical, so that a dissolution route extending from the opening 125 is further divided and compartmentalized. The insoluble portion 127 is provided around the center portion including the area around the opening 125 such that the dissolution route extends in the following order: the opening 125, between the insoluble portions 126 and 127, and between the insoluble portions 126 and 124. Because the dissolution route of the lumen passability checking capsule 120A of Modification 15 is formed longer by the compartmentalization, the time necessary for dissolution of the soluble portion 123 can be extended structurally, which maintains the domed-capsule shape for a more extended time.

In the case of the lumen passability checking capsule 120 and 120A according to the tenth embodiment and Modification 15, the soluble portion 123 forms the structure having the domed-capsule shape. Alternatively, the case where the insoluble portion 124 is provided in a thin layer such that it forms a structure having a domed-capsule shape can be similarly applied.

### (Fourth Embodiment)

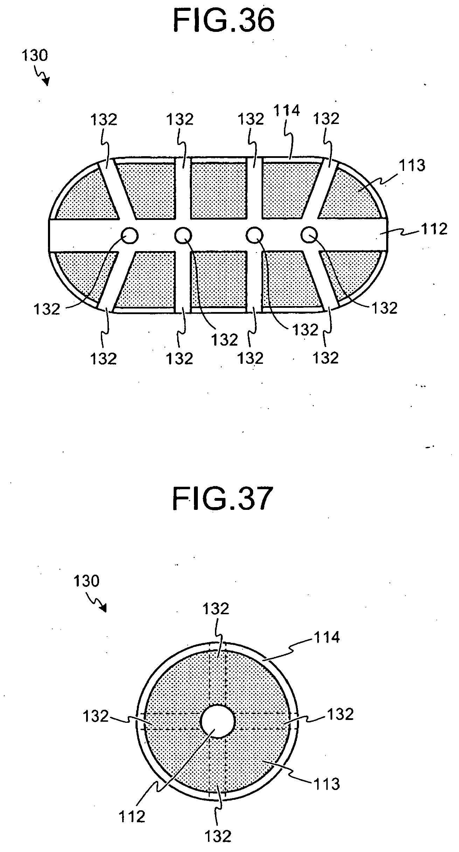

A lumen passability checking capsule according to a fourth embodiment of the present invention is explained with reference to FIGs. 36 and 37. FIG. 36 is a sectional front view of a configuration example of a lumen passability checking capsule 130 according to the fourth embodiment, and FIG. 37 is a center sectional side view of the lumen passability checking capsule 130.

In the lumen passability checking capsule 130 according to the fourth embodiment, the through hole 112 is formed such that the through hole 112 is positioned on the axial center of the soluble portion 123, which forms a structure having a domed-capsule shape, and that penetrates in the longitudinal direction, and communicating holes 132 that communicates the through hole 112 and the outside of the lumen passability checking capsule 130 with each other are radially provided from the axial center. An angle between the communicating holes 132 is, for example, 90 degrees.

In the fourth embodiment, the communicating holes 132 that communicate with the through hole 112 are further provided in addition to the through hole 112. Therefore, even if the lumen passability checking capsule 130 is stuck in various postures in, for example, a constricted portion, the portions of the lumen, which are anterior and posterior to the lumen passability checking capsule 130, can be definitely prevented from being blocked.

### (Fifth Embodiment)

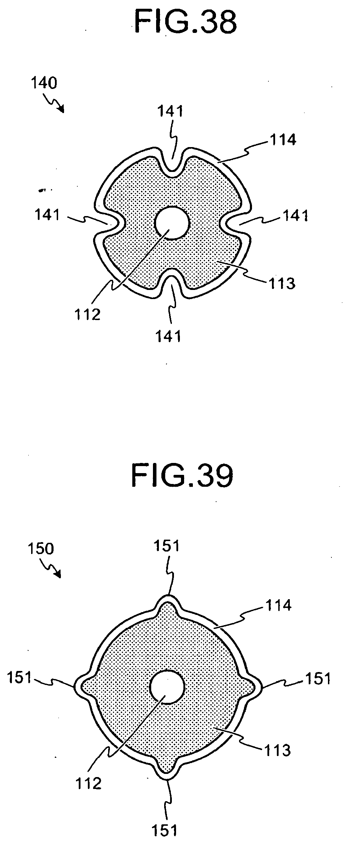

A lumen passability checking capsule 140 according to a fifth embodiment of the present invention is explained below with reference to FIG. 38. FIG. 38 is a center sectional side view of a configuration example of a lumen passability checking capsule 140 according to the fifth embodiment.

In the lumen passability checking capsule 140 according to the fifth embodiment, a plurality of grooves 141 along the longitudinal direction are provided on a surface layer of a domed-capsule shape. In the fifth embodiment, a fluid path between portions of the lumen, which are anterior and posterior to the lumen passability checking capsule 140, can be formed between the grooves 114 and a wall surface in the lumen, in addition to the through hole 112, which further prevents the lumen from being blocked.

### (Modification 16)

FIG. 39 is a center sectional side view of a lumen passability checking capsule 150 of Modification 16. In the lumen passability checking capsule 150 of Modification 16, a plurality of protruding portions 151 that extend along the longitudinal direction are provided instead of the grooves 141. Because of the protruding portions 151, a fluid path between portions of the lumen, which are anterior and posterior to the lumen passability checking capsule 150, is formed between the protruding portions 151 and the wall surface of the lumen at the bottom of the protruding portions 151, which further prevents the lumen from being blocked.

### (Sixth Embodiment)

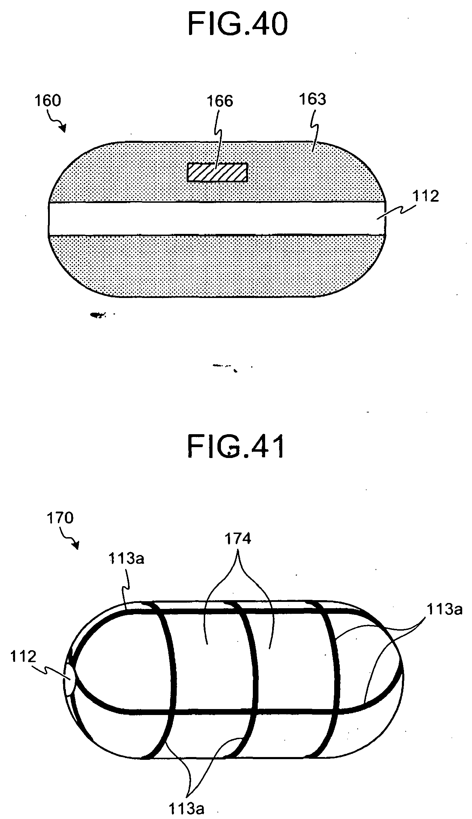

A lumen passability checking capsule according to a sixth embodiment of the present invention is explained below with reference to FIG. 40. FIG. 40 is a sectional front view of a configuration example of a lumen passability checking capsule 160 according to the sixth embodiment.

In the lumen passability checking capsule 160 according to the sixth embodiment, the through hole 112 is formed such that the through hole 112 is positioned at the axial center of a soluble portion 163, which forms a structure of a domed-capsule shape, and that penetrates in the longitudinal direction. An identified portion 166 is in the soluble portion 163, and the identified portion 166 is provided between the axial center and the surface such that the soluble portion 163 dissolves last. No insoluble portion is provided on the outer surface of the soluble portion 163. In other words, the soluble portion 163 is exposed. The rate at which the soluble portion 163 dissolves is preferably low because the domed-capsule shape can be maintained for an extended time if the dissolution rate is small. It is obvious that the dissolution of the soluble portion 163 can be started or it completely dissolves at desired timing by forming multiple layers of a plurality of materials having different dissolution rates or by mixing the materials.

In the sixth embodiment, the fluid path is always formed between portions of the lumen, which are anterior and posterior to the lumen passability checking capsule 160, by the presence of the through hole 112. Therefore, the soluble portion 163 formed of a soluble material having a low dissolution rate can be provided, which makes it possible to safely confirm a passage in the lumen with a simple configuration. It is obvious that the soluble portion 163 can contain a radiopaque member, a pigment that colors feces after dissolution, a medical drug for treatment of a damaged site, or components that has specific odor.

### (Seventh Embodiment)

A lumen passability checking capsule according to a seventh embodiment of the present invention is explained below with reference to FIG. 41. FIG. 41 is a schematic perspective view of a configuration example of a lumen passability checking capsule 170 according to the seventh embodiment.

The lumen passability checking capsule 170 according to the seventh embodiment includes the through hole 112 as in the case of the second to fifth embodiments and Modification examples 11 to 16. In addition, in the soluble portion 113, soluble portion 113a that is exposed on the surface is provided linearly in circles in the longitudinal direction and in circles in the circumferential direction on the longitudinal direction, so that the insoluble portion 114 is divided into segments of a plurality of sheet members 174. The sheet members 174 connected with the soluble portion 113a forms a structure that maintains the domed-capsule shape. Microspheres of barium or metal may be used for each of the sheet members 174 as long as substantially the same effects are achieved.

In the fourth example, when the soluble portion 113 dissolves, the insoluble portion serving as the surface can be divided into segments, which allows the insoluble portion to pass through a constricted portion more easily. The number of lines of the soluble portions 113a may be appropriately set. In addition, the longitudinal direction and the circumferential direction may be oblique, or can be combined with a spiral shape.

### (Eighth Embodiment)

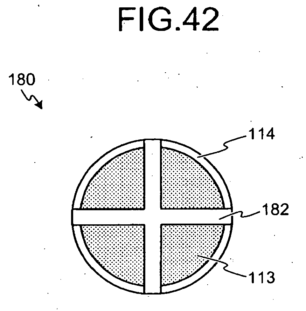

A lumen passability checking capsule according to an eighth embodiment of the present invention is explained below with reference to FIG. 42. FIG. 42 is a schematic cross section of a configuration example of a lumen passability checking capsule 180 according to the eighth embodiment.

The lumen passability checking capsule 180 according to the eighth embodiment has a spherical capsule shape. The soluble portion 113 forms a spherical structure, and the outer surface of the soluble portion 113 is coated with a film of the insoluble portion 114. In addition, a plurality of through holes 182 that pass through the center of the soluble portion 113 are provided such that the angle between the through holes 182 is an acute angle equal to or smaller than 90 degrees.

Because a fluid path between portions of the lumen, which are anterior and posterior to the lumen passability checking capsule 180, can be always formed even if the lumen passability checking capsule 180 is stuck in an constricted portion in various modes, the lumen can be definitely prevented from being blocked.

The present invention is not limited to the embodiments described above, and various modifications can be made within the scope of the invention. For example, in each of the embodiments and Modifications, a capsule body of a lumen passability checking capsule is explained, taking, as an example, the domed-capsule body having the convex portions 11a and 11b that are domed on both ends in the longitudinal direction. Alternatively, a sphere capsule body having a size approximately same as that of a capsule endoscope, which is to be used, in the lateral direction may be used. Furthermore, the insoluble portion is explained, taking the example in which it is formed of a material insoluble in the body. However, it suffices that a material relatively insoluble in the body with respect to the relationship with the soluble portion is used, and even a material soluble in the body is can be used as long as the time taken by the material to dissolve is longer than that of the material constituting the soluble portion (i.e., a material that does not easily dissolve).

### INDUSTRIAL APPLICABILITY

As explained above, the lumen passability checking device and the method of manufacturing a lumen passability checking device is useful for confirming whether a capsule-type medical device, such as a capsule endoscope, is introduced into the body of a subject to confirm beforehand whether the capsule-type medical device can pass through a lumen, before the capsule-type medical device is used, particularly, when it is used for the stomach and intestines that are hollow organs.

## Claims

1. A lumen passability checking device (50; 110; 110A; 110B; 110C; 110D; 120; 120A; 120B; 130; 140; 150; 170) that has a capsule shape and a size capable of being introduced into a body, the lumen passability checking device (50; 110; 110A; 110B; 110C; 110D; 120; 120A; 120B; 130; 140; 150; 170) comprising:
a soluble portion (41; 113; 123) that is made of a material soluble in the body, and has a capsule shape;
a through hole (51; 112; 112a to 112d; 122) penetrating between both ends of the capsule shape through the axial center of the soluble portion (41; 113; 123) in a longitudinal direction of the capsule shape; and
an insoluble portion (42; 114; 114a, 114b, 114c; 124) that is made of a material insoluble in the body, and the insoluble portion forming a film covering a surface of the soluble portion (41; 113; 123), excluding both ends of the through hole (51; 112; 112a to 112d; 122).

2. The lumen passability checking device (50; 110; 110A; 110B; 110C; 110D; 120; 120A; 120B; 130; 140; 150; 170) according to claim 1, wherein
the capsule shape is a domed-capsule shape that has convex portions on both ends in the longitudinal direction.

3. The lumen passability checking device (110D) according to claim 1, further comprising additional one or more through holes (112a to 112d) penetrating along the axial center in the longititudinal direction through the soluble portion (113).

4. The lumen passability checking device (140; 150) according to claim 1 further comprising one of a groove (141) or a protruding portion (151) that is formed along the longitudinal direction on a surface layer of the capsule shape such that a fluid path is formed between a surface of the lumen and an outer surface of the lumen passability checking device when the outer surface of the lumen passability checking device closely contacts with a wall surface of the lumen.

5. A method of manufacturing a lumen capsule confirmation device that has a capsule shape and a size capable of being introduced into a body, and that loses an original capsule shape with time because the lumen passability checking device resides in a lumen for a predetermined time or more, the method comprising:
forming a soluble portion which has a capsule shape and is soluble in the body;
forming a through hole penetrating between both ends of the capsule shape through the soluble portion in a longitudinal direction of the capsule shape; and
forming a substantially impermeable film that covers a surface of the soluble portion excluding both ends of the through hole.

6. The method according to claim 5 further comprising covering a surface of the soluble portion entirely with an enteric material.

7. The method according to claim 6, wherein the film covers a surface of the enteric material.

## Patentansprüche

1. Lumenpassierbarkeitsüberprüfungseinrichtung (50; 110; 110A; 110B; 110C; 110D; 120; 120A; 120B; 130; 140; 150; 170), die eine Kapselform und eine Größe hat, die in einen Körper eingeführt zu werden vermag, wobei die Lumenpassierbarkeitsüberprüfungseinrichtung (50; 110; 110A; 110B; 110C; 110D; 120; 120A; 120B; 130; 140; 150; 170) umfasst:
einen löslichen Abschnitt (41; 113; 123), der aus einem Material besteht, das im Körper löslich ist und eine Kapselform hat;
eine Durchgangsöffnung (51; 112; 112a bis 112d; 122), die zwischen beiden Enden der Kapselform durch den axialen Mittelpunkt des löslichen Abschnitts (41; 113; 123) in einer Längsrichtung der Kapselform hindurch geführt ist; und
einen unlöslichen Abschnitt (42; 114; 114a, 114b, 114c; 124), der aus einem Material besteht, das im Körper unlöslich ist, wobei der unlösliche Abschnitt einen Film bildet, der mit Ausnahme beider Enden der Durchgangsöffnung (51; 112; 112a bis 112d; 122) eine Oberfläche des löslichen Abschnitts abdeckt.

2. Lumenpassierbarkeitsüberprüfungseinrichtung (50; 110; 110A; 110B; 110C; 110D; 120; 120A; 120B; 130; 140; 150; 170) gemäß Anspruch 1, wobei
die Kapselform eine domförmige Kapselform ist, die konvexe Abschnitte an beiden Enden in der Längsrichtung hat.

3. Lumenpassierbarkeitsüberprüfungseinrichtung (110D) gemäß Anspruch 1, die ferner eine oder mehrere zusätzliche Durchgangsöffnung(en) (112a bis 112d) umfasst, die entlang des axialen Mittelpunkts in der Längsrichtung durch den löslichen Abschnitt (113) hindurch geführt ist/sind.

4. Lumenpassierbarkeitsüberprüfungseinrichtung (140; 150) gemäß Anspruch 1, die ferner eine Nut (141) oder einen Vorsprungabschnitt (151) umfasst, die/der entlang der Längsrichtung an einer Oberflächenschicht der Kapselform ausgebildet ist, so dass ein Fluidpfad zwischen einer Oberfläche des Lumens und einer Außenfläche der Lumenpassierbarkeitsüberprüfungseinrichtung ausgebildet ist, wenn die Außenfläche der Lumenpassierbarkeitsüberprüfungseinrichtung in engem Kontakt mit einer Wandoberfläche des Lumens steht.

5. Verfahren zur Herstellung einer Kapsel-Lumenbestätigungseinrichtung, die eine Kapselform und eine Form hat, die in einen Körper eingeführt zu werden vermag und die mit der Zeit eine ursprüngliche Kapselform verliert, da die Lumenpassierbarkeitsüberprüfungseinrichtung für eine vorbestimmte Zeit oder mehr in einem Lumen angeordnet ist, wobei das Verfahren umfasst:
Ausbilden eines löslichen Abschnitts, der eine Kapselform hat und in dem Körper löslich ist;
Ausbilden einer Durchgangsöffnung, die zwischen beiden Enden der Kapselform in einer Längsrichtung der Kapselform durch den löslichen Abschnitt hindurch geführt ist; und
Ausbilden eines im Wesentlichen undurchlässigen Films, der mit Ausnahme beider Enden der Durchgangsöffnung eine Oberfläche des löslichen Abschnitts abdeckt.

6. Verfahren gemäß Anspruch 5, das ferner ein vollständiges Abdecken einer Oberfläche löslichen Abschnitts mit einem magensaftresistenten Material umfasst.

7. Verfahren gemäß Anspruch 6, wobei der Film eine Oberfläche des magensaftresistenten Materials abdeckt.

## Revendications

1. Dispositif (50 ; 110 ; 110A ; 110B ; 110C ; 110D ; 120 ; 120A ; 120B ; 130 ; 140 ; 150 ; 170) de contrôle de possibilité de passage d'une lumière qui présente une forme de capsule et une taille capable d'être introduite dans un corps, le dispositif (50 ; 110 ; 110A ; 110B ; 110C ; 110D ; 120 ; 120A ; 120B ; 130 ; 140 ; 150 ; 170) de contrôle de possibilité de passage d'une lumière comprenant :
une partie soluble (41 ; 113 ; 123) qui est composée d'un matériau soluble dans le corps, et présente une forme de capsule ;
un trou traversant (51 ; 112 ; 112a à 112d ; 122) pénétrant entre les deux extrémités de la forme de capsule à travers le centre axial de la partie soluble (41 ; 113 ; 123) dans une direction longitudinale de la forme de capsule ; et
une partie insoluble (42 ; 114 ; 114a, 114b, 114c ; 124) qui est composée d'un matériau insoluble dans le corps, et la partie insoluble formant un film couvrant une surface de la partie soluble (41 ; 113 ; 123), excluant les deux extrémités du trou traversant (51 ; 112 ; 112a à 112d ; 122).

2. Dispositif (50 ; 110 ; 110A ; 110B ; 110C ; 110D ; 120 ; 120A ; 120B ; 130 ; 140 ; 150 ; 170) de contrôle de possibilité de passage d'une lumière selon la revendication 1, dans lequel
la forme de capsule est une forme de capsule en forme de dôme qui comporte des parties convexes sur les deux extrémités dans la direction longitudinale.

3. Dispositif (110D) de contrôle de possibilité de passage d'une lumière selon la revendication 1, comprenant en outre un ou plusieurs trous traversants (112a à 112d) supplémentaires pénétrant le long du centre axial dans la direction longitudinale à travers la partie soluble (113).

4. Dispositif (140 ; 150) de contrôle de possibilité de passage d'une lumière selon la revendication 1, comprenant en outre l'une parmi une rainure (141) ou une partie saillante (151) qui est formée le long de la direction longitudinale sur une couche de surface de la forme de capsule d'une manière telle qu'un trajet fluidique est formé entre une surface de la lumière et une surface externe du dispositif de contrôle de possibilité de passage d'une lumière lorsque la surface externe du dispositif de contrôle de possibilité de passage d'une lumière vient en contact étroit avec une surface de paroi de la lumière.

5. Procédé de fabrication d'un dispositif de confirmation de capsule de lumière qui présente une forme de capsule et une taille capable d'être introduite dans un corps, et qui perd une forme de capsule d'origine avec le temps du fait que le dispositif de contrôle de possibilité de passage d'une lumière réside dans une lumière pendant un temps prédéterminé ou plus, le procédé comprenant les étapes consistant à :
former une partie soluble qui présente une forme de capsule et est soluble dans le corps ;
former un trou traversant pénétrant entre les deux extrémités de la forme de capsule à travers la partie soluble dans une direction longitudinale de la forme de capsule ; et
former un film sensiblement imperméable qui couvre une surface de la partie soluble excluant les deux extrémités du trou traversant.

6. Procédé selon la revendication 5, comprenant en outre l'étape consistant à couvrir une surface de la partie soluble entièrement avec un matériau entérique.

7. Procédé selon la revendication 6, dans lequel le film couvre une surface du matériau entérique.
